(19) Europäisches Patentamt
European Patent Office
Office européen des brevets

(11) EP 2 762 154 A2

(12) # EUROPEAN PATENT APPLICATION

(43) Date of publication:
**06.08.2014 Bulletin 2014/32**

(51) Int Cl.:
***A61K 39/00*** *(2006.01)*

(21) Application number: **14000319.5**

(22) Date of filing: **29.01.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **05.02.2013 JP 2013020730**

(71) Applicant: **NITTO DENKO CORPORATION**
**Osaka 567 (JP)**

(72) Inventors:
- **Shishido, Takuya**
  **Ibaraki-shi**
  **Osaka 567-8680 (JP)**
- **Okubo, Katsuyuki**
  **Ibaraki-shi**
  **Osaka 567-8680 (JP)**
- **Asari, Daisuke**
  **Ibaraki-shi**
  **Osaka 567-8680 (JP)**
- **Okazaki, Arimichi**
  **Ibaraki-shi**
  **Osaka 567-8680 (JP)**
- **Maeda, Yoshiki**
  **Ibaraki-shi**
  **Osaka 567-8680 (JP)**
- **Matsushita, Kyohei**
  **Ibaraki-shi**
  **Osaka 567-8680 (JP)**
- **Li, Wenjing**
  **Ibaraki-shi**
  **Osaka 567-8680 (JP)**
- **Hori, Mitsuhiko**
  **Ibaraki-shi**
  **Osaka 567-8680 (JP)**
- **Sugiyama, Haruo**
  **Suita-shi**
  **Osaka 565-0871 (JP)**

(74) Representative: **Müller-Boré & Partner**
**Patentanwälte PartG mbB**
**Friedenheimer Brücke 21**
**80639 München (DE)**

(54) **Vaccine composition for transdermal administration**

(57) Disclosed is a vaccine composition for transdermal administration to induce cellular immunity, comprising an antigen, wherein Th1 cell ratio in a model animal for immunological evaluation that received the composition is 10% or more.

EP 2 762 154 A2

SEQUENCE LISTING

<110> NITTO DENKO CORPORATION

<120> VACCINE COMPOSITION FOR TRANSDERMAL ADMINISTRATION

<130> NITTO 1-1

<150> JP 2013-020730
<151> 2013-02-05

<160> 15

<170> PatentIn version 3.2

<210> 1
<211> 9
<212> PRT
<213> Homo sapiens

<400> 1

Ala Tyr Ala Cys Asn Thr Ser Thr Leu
1               5

<210> 2
<211> 9
<212> PRT
<213> Homo sapiens

<400> 2

Glu Tyr Ile Leu Ser Leu Glu Glu Leu
1               5

<210> 3
<211> 9
<212> PRT
<213> Homo sapiens

<400> 3

Thr Tyr Leu Pro Thr Asn Ala Ser Leu
1               5

<210> 4
<211> 9
<212> PRT
<213> Homo sapiens

<400> 4

Ile Met Pro Lys Ala Gly Leu Leu Ile
1               5

<210> 5
<211> 9
<212> PRT
<213> hepatitis C virus

<400> 5

Asp Leu Met Gly Tyr Ile Pro Ala Val
1               5

<210> 6
<211> 9
<212> PRT
<213> Homo sapiens

<400> 6

Val Leu Gln Glu Leu Asn Val Thr Val
1               5

<210> 7
<211> 9
<212> PRT
<213> Homo sapiens

<400> 7

Lys Val Phe Gly Ser Leu Ala Phe Val
1               5

<210> 8
<211> 9
<212> PRT
<213> Homo sapiens

<400> 8

Lys Val Ala Glu Ile Val His Phe Leu
1               5

<210> 9

<211> 9
<212> PRT
<213> hepatitis B virus

<400> 9

Trp Leu Ser Leu Leu Val Pro Phe Val
1               5

<210> 10
<211> 9
<212> PRT
<213> Homo sapiens

<400> 10

Lys Ile Phe Gly Ser Leu Ala Phe Leu
1               5

<210> 11
<211> 9
<212> PRT
<213> Homo sapiens

<400> 11

Ser Thr Ala Pro Pro Val His Asn Val
1               5

<210> 12
<211> 20
<212> DNA
<213> Unknown

<220>
<223> bacterial DNA

<400> 12
tccatgacgt tcctgacgtt

<210> 13
<211> 15
<212> PRT
<213> Mycobacterium tuberculosis

<400> 13

Phe Gln Asp Ala Tyr Asn Ala Ala Gly Gly His Asn Ala Val Phe

1 5 10 15

<210> 14
<211> 15
<212> PRT
<213> Artificial sequence

<220>
<223> Synthetic peptide

<400> 14

Phe Gln Asp Ala Tyr Asn Ala Val His Ala Ala His Ala Val Phe
1 5 10 15

<210> 15
<211> 13
<212> PRT
<213> Artificial sequence

<220>
<223> Synthetic peptide

<220>
<221> MOD_RES
<222> (1)..(1)
<223> D-alanine

<220>
<221> MOD_RES
<222> (3)..(3)
<223> cyclohexylalanine

<220>
<221> MOD_RES
<222> (13)..(13)
<223> D-alanine

<400> 15

Ala Lys Ala Val Ala Ala Trp Thr Leu Lys Ala Ala Ala
1 5 10

SEQUENCE LISTING

<110> NITTO DENKO CORPORATION

<120> VACCINE COMPOSITION FOR TRANSDERMAL ADMINISTRATION

<130> NITTO 1-1

<150> JP 2013-020730
<151> 2013-02-05

<160> 15

<170> PatentIn version 3.2

<210> 1
<211> 9
<212> PRT
<213> Homo sapiens

<400> 1

Ala Tyr Ala Cys Asn Thr Ser Thr Leu
1               5

<210> 2
<211> 9
<212> PRT
<213> Homo sapiens

<400> 2

Glu Tyr Ile Leu Ser Leu Glu Glu Leu
1               5

<210> 3
<211> 9
<212> PRT
<213> Homo sapiens

<400> 3

Thr Tyr Leu Pro Thr Asn Ala Ser Leu
1               5

<210> 4
<211> 9
<212> PRT
<213> Homo sapiens

<400> 4

Ile Met Pro Lys Ala Gly Leu Leu Ile
1               5

<210> 5
<211> 9
<212> PRT
<213> hepatitis C virus

<400> 5

Asp Leu Met Gly Tyr Ile Pro Ala Val
1 5

<210> 6
<211> 9
<212> PRT
<213> Homo sapiens

<400> 6

Val Leu Gln Glu Leu Asn Val Thr Val
1 5

<210> 7
<211> 9
<212> PRT
<213> Homo sapiens

<400> 7

Lys Val Phe Gly Ser Leu Ala Phe Val
1 5

<210> 8
<211> 9
<212> PRT
<213> Homo sapiens

<400> 8

Lys Val Ala Glu Ile Val His Phe Leu
1 5

<210> 9
<211> 9
<212> PRT
<213> hepatitis B virus

<400> 9

Trp Leu Ser Leu Leu Val Pro Phe Val
1 5

<210> 10
<211> 9
<212> PRT
<213> Homo sapiens

<400> 10

Lys Ile Phe Gly Ser Leu Ala Phe Leu
1 5

<210> 11

<211> 9
<212> PRT
<213> Homo sapiens

<400> 11

Ser Thr Ala Pro Pro Val His Asn Val
1 5

<210> 12
<211> 20
<212> DNA
<213> Unknown

<220>
<223> bacterial DNA

<400> 12
tccatgacgt tcctgacgtt 20

<210> 13
<211> 15
<212> PRT
<213> Mycobacterium tuberculosis

<400> 13

Phe Gln Asp Ala Tyr Asn Ala Ala Gly Gly His Asn Ala Val Phe
1 5 10 15

<210> 14
<211> 15
<212> PRT
<213> Artificial sequence

<220>
<223> Synthetic peptide

<400> 14

Phe Gln Asp Ala Tyr Asn Ala Val His Ala Ala His Ala Val Phe
1 5 10 15

<210> 15
<211> 13
<212> PRT
<213> Artificial sequence

<220>
<223> Synthetic peptide

<220>
<221> MOD_RES
<222> (1)..(1)
<223> D-alanine

<220>
<221> MOD_RES

```
<222>  (3)..(3)
<223>  cyclohexylalanine

<220>
<221>  MOD_RES
<222>  (13)..(13)
<223>  D-alanine

<400>  15

Ala Lys Ala Val Ala Ala Trp Thr Leu Lys Ala Ala Ala
1               5                   10
```

**Description**

TECHNICAL FIELD

The present invention relates to a vaccine composition for transdermally inducing cellular immunity.

BACKGROUND ART

Vaccines are widely used in order to induce immunity into the subject and include those for administering pathogens such as microorganisms or viruses, or a part thereof. There is a cancer vaccine for allowing a cellular immunity mechanism to recognize a cancer cell specific antigen and inducing a specific attack of the immune system to cancer cells, which is used as one measure for treating a cancer.

In usual, the invasion of microorganisms and viruses into the bio-body is prevented by skin due to the size thereof, and it is necessary to invasively administrate a vaccine into the bio-body. Accordingly, injections are usually used in order to provide immunity. Injections, however, have problems of pain, fear, an injection scar, and a needle mark and scarring thereof, and have further problems that only a medical worker is allowed to perform such administration; it is technically difficult to perform an intradermal injection having a high immune effect; there is a risk such as an infection accident caused by needlestick by a medical worker; patients are forced to go repeatedly to the hospital when repeated injection is required; and it causes medical wastes such as injection needle which is required to be disposed by a special method. Thus, injection is not necessarily an optimal route of administration.

Subcutaneous injection or intradermal injection is most generally used as the route of administration of a vaccine, but in addition to them, various routes of administration have been tried to induce immunity, for example, transdermal administration (Patent Document 1 and Non-Patent Document 1), buccal administration, transnasal administration, and sublingual administration (Non-Patent Document 2, Patent Document 2 and Patent Document 3).

In order to provide immunity by injection, it is usually used an adjuvant. For example, aluminum salts such as aluminum hydroxide, aluminum phosphate and aluminum chloride, and emulsions including squalene such as MF59 and AS03 are practically used as an adjuvant, and in addition to them, flagellum components, nucleic acids, cytokines, cationic polymers and polypeptides are widely studied as an adjuvant. With respect to an adjuvant to be used for other route than injection such as transdermal administration or transmucosal administration to provide immunity, it has also been studied to use a substance such as aluminum salts (e.g. aluminum hydroxide, aluminum phosphate and aluminum chloride), and toxins (e.g. cholera toxin and heat-labile E. coli toxin), but they have not yet been put into practical use. Most of them are used as an adjuvant for inducing humoral immunity by producing antibodies to prevent infection from viruses or bacteria. On the other hand, as for only cellular immunity induction, a Freund adjuvant, Montanide, GM-CSF, IL-2, IL-12 and IFN-$\gamma$ have been studied as an adjuvant for injection, but they have still not yet been put into practical use. Besides, in the route of transdermal administration or mucosal administration, there are only a few reports about toxins such as cholera toxin and heat-labile E. coli toxin, and nucleic acids.

LIST OF DOCUMENTS

[Patent Document 1] US-A-2008/0193487
[Patent Document 2] JP-A-2002-531415
[Patent Document 3] US-A-2008/0112974
[Patent Document 4] JP-A-7-505883
[Patent Document 5] JP-A-2007-529531

[Non-Patent Document 1] Hosoi Akihiro et al., Cancer Research, 68, 3941-3949 (2008)
[Non-Patent Document 2] Zhengrong Cui et al., Pharmaceutical Research, Vol.19, No.7, 947-953 (2002)

SUMMARY OF THE INVENTION

Transdermal administration has been thought as one measure for solving various problems regarding injection. However, an effective cellular immunity induction promoter which can be used in the cellular immunity induction by transdermal administration of an antigen has been poorly reported, and in many cases, a sufficient cellular immunity induction cannot be obtained by transdermal administration as compared with injection.

An object of the present invention is to provide a vaccine composition which exerts a high cellular immunity inducing effect by transdermal administration.

The present invention provides a vaccine composition which comprises a cellular immunity induction promoter and exerts a high cellular immunity inducing effect by transdermal administration, wherein the cellular immunity induction promoter can be used for transdermal administration of various antigens to induce cellular immunity.

The present inventors have focused on helper T cells, particularly a balance between the number of Th1 cells and the number of Th2 cells, among various cells which are involved in cellular immunity.

Regarding the immunity induction through transdermal administration, in order to increase antigen permeability, a pre-treatment of skin, for example, removal of corneum by a tape-stripping, is generally performed. The present inventors, however, have found that the irritation of skin caused by such a pre-treatment increases a ratio of the number of the Th2 cells to the total number of the Th1 cells and the Th2 cells, resulting in the reduction in cellular immunity inducing effect, whereas under a condition where irritation of the skin is mild, the present inventors have found that the ratio of the number of the Th1 cells to the total number of the Th1 cells and the Th2 cells is increased and the cellular immunity inducing effect is increased. They have further found that addition of an additive which increases the number of the Th1 cells causes the ratio of the Th1 cell to be more increased and causes the cellular immunity inducing effect to be more increased, as compared with the case where irritation of the skin is mild. They have found, accordingly, that when the ratio of the Th1 cells is increased as for the balance between the Th1 cell and the Th2 cell in a subject, an increased cellular immunity inducing effect can be obtained by transdermal administration of a desired antigen. They have further found that the cellular immunity inducing effect can be obtained by the transdermal administration of the vaccine composition of the present invention, which is found to be more increased than that obtained by injection administration.

In one aspect of the present invention, the cellular immunity induction obtained by transdermal administration of an antigen is potentiated by using a specific cellular immunity induction promoter together with the antigen. Specifically, when one or more cellular immunity induction promoters selected from the group consisting of a TLR ligand, a cyclic dinucleotide, a helper peptide and an immunomodulatory small molecule drug are used in a vaccine composition for transdermal administration, a high cellular immunity inducing effect can be obtained by the transdermal administration.

In one embodiment of the present invention, the induction of cellular immunity by transdermal administration of an antigen is enhanced by administering the antigen under a mildly irritating condition. Specifically, the high cellular immunity inducing effect is obtained by selecting the mildly irritating state where transepidermal water loss (TEWL) ($g/h \cdot m^2$), which is an index of the state of the skin of a model animal for skin irritation evaluation, before the administration of the vaccine composition for transdermal administration is 50 or less, and administering transdermally the vaccine composition for transdermal administration. Alternatively, a higher cellular immunity inducing effect can be obtained when the vaccine composition for transdermal administration has such a mildly irritating property that the cutaneous TSLP level (pg/mg protein) of the model animal for skin irritation evaluation at completion of the administration of the composition to the animal is 10000 or less.

In one aspect of the present invention, the balance between the Th1 cell and the Th2 cell is controlled by using a specific cellular immunity induction promoter together with an antigen. Specifically, when one or more cellular immunity induction promoters selected from the group consisting of a TLR ligand, a cyclic dinucleotide, a helper peptide and an immunomodulatory small molecule drug are used in the vaccine composition for transdermal administration, the ratio of Th1 cells/(Th1 cells + Th2 cells) can be increased. As a result, a high cellular immunity inducing effect can be obtained by the transdermal administration.

In one aspect of the present invention, the balance between the Th1 cell and the Th2 cell is also controlled by administration of a vaccine composition of the present invention in a mildly-irritating condition. Specifically, a vaccine composition for transdermal administration is administered under a mildly-irritating condition where a transepidermal water loss (TEWL) ($g/h \cdot m^2$) which is an indicator of the skin state of a model animal for skin irritation evaluation before the administration of the vaccine composition for transdermal administration is 50 or less, the ratio of Th1 cells/ (Th1 cells + Th2 cells) can be increased; and as a result, a high cellular immunity inducing effect can be obtained. Alternatively, also when a vaccine composition for transdermal administration is made to be mildly-irritating so that a cutaneous TSLP level (pg/mg protein) at the end of administration is 10000 or less, the ratio of the Th1 cells/ (Th1 cells + Th2 cells) can be increased; and as a result, a high cellular immunity inducing effect can be obtained.

The present invention, accordingly, provides embodiments listed below.

(1) A vaccine composition for transdermal administration to induce cellular immunity, comprising an antigen, wherein a Th1 cell ratio in a model animal, administered with the composition, for immunological evaluation is 10% or more;
(2) A vaccine composition for transdermal administration to induce cellular immunity, wherein the composition comprises an antigen and one or more cellular immunity induction promoters selected from the group consisting of a TLR ligand, a cyclic dinucleotide, a helper peptide and an immunomodulatory small molecule drug;
(3) The vaccine composition according to (1) or (2), wherein the composition comprises one or more cellular immunity induction promoters selected from the group consisting of a TLR ligand, a cyclic dinucleotide, a helper peptide and an immunomodulatory small molecule drug, and thus the Th1 cell ratio in the model animal for immunological

evaluation is 10% or more;

(4) The vaccine composition according to (2) or (3), wherein the cellular immunity induction promoter is a TLR ligand;

(5) The vaccine composition according to (2) or (3), wherein the cellular immunity induction promoter is a cyclic dinucleotide;

(6) The vaccine composition according to (2) or (3), wherein the cellular immunity induction promoter is an immunomodulatory small molecule drug;

(7) The vaccine composition according to (2) or (3), wherein the cellular immunity induction promoter is a helper peptide;

(8) The vaccine composition according to (2) or (3), wherein the cellular immunity induction promoter is a combination of one or more selected from the group consisting of a TLR ligand, a cyclic dinucleotide and an immunomodulatory small molecule drug, and a helper peptide;

(9) The vaccine composition according to any one of (1) to (8), wherein the composition is administered in a mildly-irritating condition;

(10) The vaccine composition according to (9), wherein the mildly-irritating condition is a condition in which a transepidermal water loss (TEWL) is found to be 50 g/h•$m^2$ or less before administration in a model animal for skin irritation evaluation;

(11) The vaccine composition according to (9) or (10), wherein the mildly-irritatirig condition is a condition in which a cutaneous TSLP level is found to be10000 pg/mg skin protein or less at the end of administration in a model animal for skin irritation evaluation; and

(12) The vaccine composition according to any one of (1) to (11), wherein the antigen is a peptide selected from the group consisting of survivin-2B peptide and/or modified survivin-2B peptide, GPC3 peptide and/or modified GPC3 peptide, HER2/neu_A24 peptide and/or modified HER2/neu_A24 peptide, MAGE3_A24 peptide and/or modified MAGE3_A24 peptide, IPEP87 peptide and/or modified IPEP87 peptide, PR1 peptide and/or modified PR1 peptide, HER2/neu_A02 peptide and/or modified HER2/neu_A02 peptide, MAGE3_A02 peptide and/or modified MAGE3_A02 peptide, HBVenv peptide and/or modified HBVenv peptide, and MUC1 peptide and/or modified MUC1 peptide.

In another aspect, the vaccine composition of the present invention can be used in treatment or prevention of diseases. The present invention, accordingly, also provides embodiments listed below.

(13) A method for treating or preventing a cancer comprising: transdermally administrating to a subject a therapeutically effective amount of (i) a cancer antigen, and (ii) one or more cellular immunity induction promoters selected from the group consisting of a TLR ligand, a cyclic dinucleotide, a helper peptide and an immunomodulatory small molecule drug;

(14) The method according to (13), wherein the cancer antigen is a cancer antigen peptide selected from the group consisting of survivin-2B peptide and/or modified survivin-2B peptide, GPC3 peptide and/or modified GPC3 peptide, HER2/neu_A24 peptide and/or modified HER2/neu_A24 peptide, MAGE3_A24 peptide and/or modified MAGE3_A24 peptide, PR1 peptide and/or modified PR1 peptide, HER2/neu_A02 peptide and/or modified HER2/neu_A02 peptide, MAGE3_A02 peptide and/or modified MAGE3_A02 peptide, and MUC1 peptide and/or modified MUC1 peptide;

(15) A method for treating or preventing a viral disease comprising: transdermally administrating to a subject a therapeutically effective amount of (i) a virus antigen, and (ii) one or more cellular immunity induction promoters selected from the group consisting of a TLR ligand, a cyclic dinucleotide, a helper peptide and an immunomodulatory small molecule drug; and

(16) The method according to (15), wherein the virus antigen is a peptide selected from the group consisting of IPEP87 peptide and/or modified IPEP87 peptide, and HBVenv peptide and/or modified HBVenv peptide.

In another aspect, the present invention provides a TLR ligand, a cyclic dinucleotide, a helper peptide, an immunomodulatory small molecule drug, or a mixture of two or more thereof, for use as a cellular immunity induction promoter for transdermal administration of an antigen. The present invention also provides the following embodiments:

(17) A TLR ligand, a cyclic dinucleotide, a helper peptide, an immunomodulatory small molecule drug, or a combination of two or more thereof, for use as a cellular immunity induction promoter when an antigen is transdermally administered.

The present invention also provides the following embodiments:

(18) A method of inducing cellular immunity, which comprises transdermally administering to a subject an antigen,

and one or more cellular immunity induction promoters selected from the group consisting of TLR ligand, cyclic dinucleotide, helper peptide and immunomodulatory small molecule drug;

(19) TLR ligand, cyclic dinucleotide, helper peptide, immunomodulatory small molecule drug or a combination of two or more of them, for use in promoting the induction of cellular immunity by the transdermal administration of an antigen;

(20) A combination of an antigen and one or more cellular immunity induction promoters selected from the group consisting of TLR ligand, cyclic dinucleotide, helper peptide and immunomodulatory small molecule drug, for use in inducing cellular immunity by the transdermal administration ,of an antigen;

(21) A combination of (i) a cancer antigen and (ii) one or more cellular immunity induction promoters selected from the group consisting of TLR ligand, cyclic dinucleotide, helper peptide and immunomodulatory small molecule drug for use in treating or preventing a cancer, wherein the combination is transdermally administered;

(22) A combination of (i)a virus antigen and (ii) one or more cellular immunity induction promoters selected from the group consisting of TLR ligand, cyclic dinucleotide, helper peptide and immunomodulatory small molecule drug for use in treating or preventing a viral disease, wherein the combination is transdermally administered;

(23) Use of an antigen and one or more cellular immunity induction promoters selected from the group consisting of TLR ligand, cyclic dinucleotide, helper peptide and immunomodulatory small molecule drug, for the manufacture of vaccine composition for transdermal administration intended for the induction of cellular immunity;

(24) Use of (i) a cancer antigen and (ii) one or more cellular immunity induction promoters selected from the group consisting of TLR ligand, cyclic dinucleotide, helper peptide and immunomodulatory small molecule drug, for the manufacture of vaccine composition for transdermal administration for the treatment or prevention of a cancer; and

(25) Use of (i)a virus antigen and (ii) one or more cellular immunity induction promoters selected from the group consisting of TLR ligand, cyclic dinucleotide, helper peptide and immunomodulatory small molecule drug, for the manufacture of vaccine composition for transdermal administration for the treatment or prevention of a viral disease.

The vaccine composition of the present invention can be transdermally administered, and thus excellent compliance is achieved. For example, the vaccine composition of the present invention has the advantages that: the composition can be non-invasively administered;

the composition can be painlessly administered; fear of injection can be removed from the patient; the composition can be administered by a patient himself/herself because of ease of administration;

a risk of an infection accident caused by needlestick injury of a medical worker can be avoided;

the frequency of hospital visit when repeated administrations are performed can be reduced resulting in contribution to improvement of quality of life of the patient; and

medical waste such as an injection needle is not generated. In addition, when the composition is in the form of adhesive skin patch such as a poultice preparation or a tape preparation, the composition has advantages that a predetermined dosage can be surely administered, drug-releasing speed can be arbitrarily controlled, and the composition does not adhere to other sites on administration. Further, the adhesive skin patch can be easily attached and detached, and thus the composition has also an advantage that a patient himself/herself can immediately stop the administration by removing the adhesive skin patch from the applied site, when a side-effect occurs. Furthermore, in one preferred embodiment, the composition has also an advantage that the effect of the vaccine composition of the present invention is remarkably improved as compared to the case of single administration of an antigen. Furthermore, in one preferred embodiment, the composition has also an advantage that the transdermal administration of the vaccine composition of the present invention can induce stronger immunity than the injection administration thereof.

## DETAILED DESCRIPTION OF THE INVENTION

In order to more easily understand the present invention, the terms as used herein are defined as described below. The terms having no definition have meanings generally understood by those skilled in the art, particularly in the fields of medical science, pharmacy, immunology, cell biology, biochemistry, and polymer chemistry, unless otherwise particularly indicated in the context.

### I. Definition

The term "antigen" as used herein means any substance capable of inducing an immune response, and examples include proteins and peptides. For the transdermal administration in which cutaneous permeability of an antigen is required, an antigen having a small molecular weight is preferably used, and for example, a peptide consisting of about 8 to about 12 amino acids can be used. In the present invention, for example, the following peptides: survivin-2B peptide, GPC3 peptide, HER2/neu_A24 peptide, MAGE3_A24 peptide, IPEP87 peptide, PR1 peptide, HER2/neu_A02 peptide, MAGE3_A02 peptide, HBVenv peptide, HER2/neu E75 peptide, and MUC1 peptide can be used as the antigen.

In one embodiment, one or more peptides selected from the group consisting of HER2/neu E75 peptide for cancer vaccine applications, modified HER2/neu E75 peptide for cancer vaccine applications, WT1 peptide for cancer vaccine applications, and modified WT1 peptide for cancer vaccine applications are excluded from the antigen for use in the vaccine composition of the present invention.

The term "survivin-2B peptide" as used herein means a peptide derived from a cancer gene product, survivin, consisting of a sequence: Ala Tyr Ala Cys Asn Thr Ser Thr Leu (SEQ NO: 1)

The term "GPC3 peptide" as used herein means a peptide derived from a cancer gene product, GPC3, consisting of a sequence: Glu Tyr Ile Leu Ser Leu Glu Glu Leu (SEQ NO: 2).

The term "HER2/neu_A24 peptide" as used herein means an HLA-A24-restricted peptide derived from a cancer gene product, HER2/neu, consisting of a sequence: Thr Tyr Leu Pro Thr Asn Ala Ser Leu (SEQ NO: 3).

The term "MAGE3_A24 peptide" as used herein means an HLA-A24-restricted peptide derived from a cancer gene product, MAGE3, consisting of a sequence: Ile Met Pro Lys Ala Gly Leu Leu Ile (SEQ NO: 4).

The term "IPEP87 peptide" as used herein means a peptide derived from hepatitis C virus (HCV) protein, consisting of a sequence: Asp Leu Met Gly Tyr Ile Pro Ala Val (SEQ NO: 5).

The term "PR1 peptide" as used herein means a peptide derived from a cancer gene product, proteinase-3, consisting of a sequence: Val Leu Gln Glu Leu Asn Val Thr Val (SEQ NO: 6).

The term "HER2/neu_A02 peptide" as used herein means an HLA-A02-restricted peptide derived from a cancer gene product, HER2/neu, consisting of a sequence: Lys Val Phe Gly Ser Leu Ala Phe Val (SEQ NO: 7).

The term "MAGE3_A02 peptide" as used herein means an HLA-A02-restricted peptide derived from a cancer gene product, MAGE3, consisting of a sequence: Lys Val Ala Glu Ile Val His Phe Leu (SEQ NO: 8).

The term "HBVenv peptide" as used herein means a peptide derived from hepatitis B virus (HBV)protein, consisting of a sequence: Trp Leu Ser Leu Leu Val Pro Phe Val (SEQ NO: 9).

The term "HER2/neu E75 peptide" as used herein means a peptide derived from a product of cancer gene, HER2/neu (HER2 protein), consisting of a sequence: Lys Ile Phe Gly Ser Leu Ala Phe Leu (SEQ NO: 10).

The term "MUC1 peptide" as used herein means a peptide derived from MUC1 protein, which is a glycoprotein highly expressed on many cancer cells, consisting of a sequence: Ser Thr Ala Pro Pro Val His Asn Val (SEQ NO: 11).

The term "WT1 peptide" as used herein means a partial peptide consisting of about 8 to about 15, preferably about 8 to about 12, amino acids. WT1 peptide is a peptide obtained by fragmenting the WT1 protein which is a product of cancer gene WT1 (Wilm's tumor 1), and includes Db126 peptide and Db235 peptide (both are described in Japanese Patent No. 4422903). In addition, a partial peptide of WT1 product disclosed in WO 2000/06602, HLA-A26 binding cancer antigen peptide derived from WT1 described in WO 2005/095598, HLA-A* 3303-restricted WT1 peptide described in WO 2007/097358, and HLA-A* 1101-restricted WT1 peptide described in WO 2008/081701 are also included in the "WT1 peptide" described herein.

The term "modified XX peptide" (XX can be any peptide) as used herein means a modified peptide in which all or a part of amino acids of the XX peptide are substituted or modified.

Examples of the modified XX peptide include:

(a) a peptide consisting of an amino acid sequence in which one to several, for example, 1, 2, 3, 4 or 5 amino acids are substituted, deleted or added in the amino acid sequence of the XX peptide; and

(b) a peptide consisting of an amino acid sequence in which all or a part of amino acids, for example, one or several, such as 1, 2, 3, 4, 5, 6, 7, 8, 9, or 10 amino acids are modified in the amino acid sequence of the XX peptide.

Examples of the "modification" in the amino acid which may occur in the modified XX peptide includes, but are not limited to, acetylation, alkylation such as methylation, glycosylation, hydroxylation, carboxylation, aldehydation, phosphorylation, sulfonylation, formylation, modification by addition of an aliphatic chain such as myristoylation, palmitoylation or stearoylation, octanoylation, esterification, amidation, deamidation, modification by disulfide bond formation such as cystine modification, glutathione modification or thioglycolic acid modification, glycation, ubiquitination, succinimide formation, glutamylation, and prenylation. The modified XX peptide may contain one or more amino acids substituted, deleted or added in combination with one or more amino acids modified.

In a preferred embodiment, the antigen contained in the vaccine composition for transdermal administration of the present invention is a peptide selected from the group consisting of survivin-2B peptide and/or modified survivin-2B peptide, GPC3 peptide and/or modified GPC3 peptide, HER2/neu_A24 peptide and/or modified HER2/neu_A24 peptide, MAGE3_A24 peptide and/or modified MAGE3_A24 peptide, IPEP87 peptide and/or modified IPEP87 peptide, PR1 peptide and/or modified PR1 peptide, HER2/neu_A02 peptide and/or modified HER2/neu_A02 peptide, MAGE3_A02 peptide and/or modified MAGE3_A02 peptide, HBVenv peptide and/or modified HBVenv peptide, and MUC1 peptide and/or modified MUC1 peptide. Alternatively, HER2/neu E75 peptide and/or modified HER2/neu E75 peptide may be used as the antigen.

The peptide listed above can be free or in the form of any pharmacologically acceptable salt, for example, acid

salts (acetate, TFA salt, hydrochloride, sulfate, phosphate, lactate, tartrate, maleate, fumarate, oxalate, hydrobromide, succinate, nitrate, malate, citrate, oleate, palmitate, propionate, formate, benzoate, picrate, benzenesulfonate, dodecylsulfate, methanesulfonate, p-toluenesulfonate, glutarate, various amino acid salts, and the like), metal salts (alkali metal salts (such as sodium salt and potassium salt), alkaline earth metal salts (such as calcium salt and magnesium salt), aluminum salts, and the like), and amine salts (triethylamine salt, benzylamine salt, diethanolamine salt, t-butylamine salt, dicyclohexylamine salt, arginine salt, dimethylammonium salt, ammonium salt, and the like). The pharmacologically acceptable salt is preferably acetate or a TFA salt. The peptide described above, which can be used as the antigen in the present invention, is synthesized or produced by a well-known method, and the peptide isolated therefrom and then purified can be used.

The terms "Th1 cell" and "Th2 cell" as used herein mean type 1 helper T cell and type 2 helper T cell, respectively.

The term."Th1 cell ratio" as used herein means a ratio (percentage) of the number of Th1 cells to the total number of Th1 cells and Th2 cells, calculated by the following formula:

$$\text{Th1 cell ratio (\%)} = [\text{the number of Th1 cells}/(\text{the number of Th1 cells} + \text{the number of Th2 cells})] \times 100$$

The number of Th1 cells and the number of Th2 cells can be measured according to an immunity induction experiment using a model animal for immunological evaluation, and the ELISPOT method (IFN-γ, IL-4).

In one embodiment of the present invention, the vaccine composition of the present invention is characterized in that the Th1 cell ratio in a model animal, administered with the composition, for immunological evaluation is more than 10%. The Th1 cell ratio in a model animal, administered with the vaccine composition of the present invention, for immunological evaluation can be, for example, 10% or more, 15% or more, 20% or more, 25% or more, 30% or more, 35% or more, 40% or more, 45% or more, 50% or more, 55% or more, 60% or more, 65% or more, 70% or more, 75% or more, or 80% or more. In a case where the vaccine composition comprises HLA-A* 24 MHC-restricted class 1 peptide, when BALB/c-mice, which are model animals for immunological evaluation, are evaluated, the Th1 cell ratio after the administration of the composition is preferably 10% or more, for example, 15% or more, 20% or more, or25% or more; more preferably 30% or more, for example, 35% or more, 40% or more, or 45% or more; further more preferably 50% or more, for example, 55% or more, 60% or more, 65% or more, 70% or more, 75% or more, or 80% or more. In a case where the vaccine composition comprisesHLA-A* 02 MHC-restricted class 1 peptide, when genetically modified mice which allow the evaluation of the cellular immunity induction by HLA-A* 02 MHC-restricted peptide are used as model animals for immunological evaluation, the Th1 cell ratio after the administration of the composition is preferably 10% or more, for example, 15% or more, 20% or more, or 25% or more; more preferably 30% or more, for example, 35% or more, 40% or more, or 45% or more; further more preferably 50% or more, for example, 55% or more, 60% or more, 65% or more, 70% or more, 75% or more, or 80% or more. In the most preferred embodiment, the Th1 cell ratio in the model animal, administered with the vaccine composition of the present invention, for immunological evaluation is 10% or more when the vaccine composition comprising HLA-A* 24 MHC-restricted class 1 peptide is administered to BALB/c-mice, and the Th1 cell ratio is 50% or more when the vaccine composition comprising HLA-A* 02 MHC-restricted class 1 peptide is administered to genetically modified mice which allow the evaluation of the cellular immunity induction by HLA-A* 02 MHC-restricted peptide.

When the vaccine composition of the present invention which achieves a Th1 cell ratio of 10% or more in the model animal for immunological evaluation is administered to a desired subject, for example, human, it increases the Th1 cell ratio in the subject to exhibit a high cellular immunity inducing effect.

In one preferred embodiment, the Th1 cell ratio of 10% or more in the model animal for immunological evaluation can be attained by administration of a vaccine composition comprising an antigen and one or more cellular immunity induction promoters selected from the group consisting of a TLR ligand, a cyclic dinucleotide, a helper peptide and an immunomodulatory small molecule drug.

The Th1 cell ratio in the model animal, administered with the vaccine composition of the present invention, for immunological evaluation may be measured after 6 days from the final administration of the composition. A sample for measurement of the Th1 cell ratio may be spleen of the model animal for immunological evaluation.

The term "cellular immunity induction promoter" as used herein means any substance which can improve an efficiency of inducing a cellular immunity of an antigen, which is co-administrated together with the promoter, as compared with the efficiency obtained by administration with no promoter, and which is not limited by a mechanism to promote the cellular immunity induction.

The term "TLR ligand" as used herein means a Toll-like receptor (TLR) ligand, and examples include TLR1 to TLR9 ligands. The TLR ligand includes TLR1/2 ligand, TLR2/6 ligand, TLR2 and Dectin 1 ligand, TLR3 ligand, TLR4 ligand, TLR5 ligand, TLR7 and/or TLR8 ligand, and TLR9 ligand. In a preferred embodiment of the present invention,

the TLR ligand are TLR1/2 ligand, TLR2 and Dectin 1 ligand, TLR3 ligand, TLR4 ligand, TLR7 and/or TLR8 ligand, and/or TLR9 ligand.

The term "TLR1/2 ligand" as used herein means a ligand of a heterodimer of Toll-like receptor (TLR) 1 and Toll-like receptor (TLR) 2. Examples include triacylated lipoprotein derived from a cell wall of bacterium and a salt thereof, which may be an extract, a product or a synthesized product, but are not limited thereto.

In a preferred embodiment of the present invention, the TLR1/2 ligand is $Pam_3CSK_4$. $Pam_3CSK_4$ has the formula:

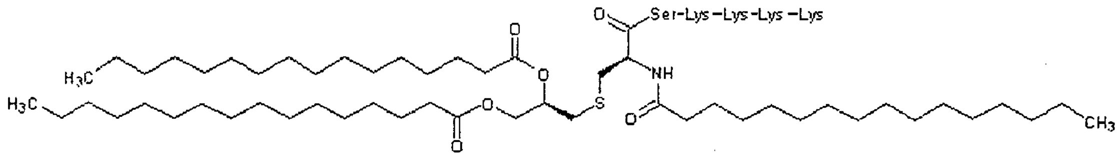

The term "TLR2 and Dectin 1 ligand" as used herein means a ligand of a heterodimer of Toll-like receptor (TLR) 2 and β1,3-glucan receptor (Dectin 1). Examples include β1,3-glucan derived from a cell wall of fungus, and a salt thereof, which may be an extract, a product, or a synthesized product, but are not limited thereto. In a preferred embodiment of the present invention, the TLR2 and Dectin 1 ligand is Zymosan derived from a cell wall of yeast.

The term "TLR3 ligand" as used herein means a ligand of Toll-like receptor (TLR) 3. Examples include double stranded RNA (dsRNA) derived from virus, and a salt thereof, which may be an extract, a product, or a synthesized product, but are not limited thereto. In a preferred embodiment of the present invention, the TLR3 ligand is polyinosinic-polycytidylic acid (Poly (I:C)) which is a synthetic product, and/or a salt thereof.

The term "TLR4 ligand" as used herein means a ligand of Toll-like receptor (TLR) 4. Examples include, but are not limited to, a lipopolysaccharide (LPS) derived from bacteria or plant, in particular, lipid A derivatives such as monophosphoryl lipid A, 3-deacylated monophosphoryl lipid A (3D-MPL), OM 174, OM 294 DP or OM 197 MP-Ac DP; alkyl glucosaminide phosphate (AGP), for example, AGP disclosed in WO 9850399 or US 6303347, or a salt of AGP disclosed in US 6764840, lipopolysaccharide derived from Pantoea bacterium, glucopyranosyl lipid, and sodium hyaluronate.

In a preferred embodiment of the present invention, the TLR4 ligand is a lipopolysaccharide derived from the genus Acetobacter (for example, Acetobacter aceti, Acetobacter xylinum, and Acetobacter orientalis), the genus Zymomonas (for example, Zymomonas mobilis), the genus Xanthomonas (for example, Xanthomonas campestris), the genus Enterobacter (for example, Enterobacter cloacae), or the genus Pantoea (for example, Pantoea agglomerans). It is possible to use the extract derived from the lipopolysaccharide or purified lipopolysaccharide as it is. In addition, for example, lipopolysaccharide (IP-PA1) derived from Pantoea agglomerans is available from Funakoshi Corporation. In a preferred embodiment of the present invention, the TLR4 ligand is lipopolysaccharide derived from Pantoea bacterium, glucopyranosyl lipid and/or sodium hyaluronate.

The term "TLR7 and/or TLR8 ligand" as used herein means a ligand of Toll-like receptor (TLR) 7 and/or TLR 8. Examples include, but is not limited to, single stranded RNA, imiquimod, resiquimod (R848), TLR7-II, and other compounds such as loxoribine and bropirimine.

In a preferred embodiment of the present invention, the TLR 7 and/or TLR 8 ligand is imiquimod. The imiquimod is 1-(2-methylpropyl)-1H-imidazo[4,5-c]quinolin-4-amine of the formula:

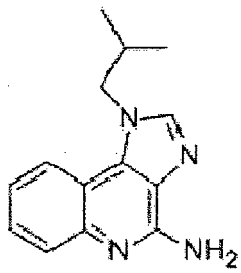

characteristics and production method of which are described, for example, in JP-A-7-505883 (Patent Document 4).

In another preferred embodiment, the TLR7 and/or TLR8 ligand is resiquimod. The resiquimod is 4-amino-2-(ethoxymethyl)-a,a-dimethyl-1H-imidazo[4,5-c]qui nolin-a-ethanol of the formula:

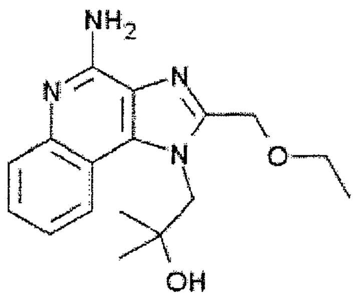

In another preferred embodiment, the TLR7 and/or TLR8 ligand is TLR7-II. The TLR7-II is represented by the formula:

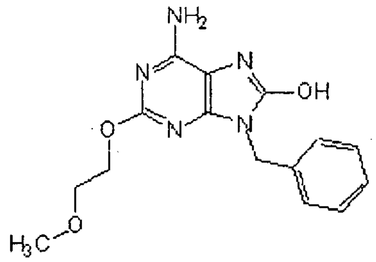

In another preferred embodiment, the TLR7 and/or TLR8 ligand is bropirimine. The bropirimine is represented by the formula:

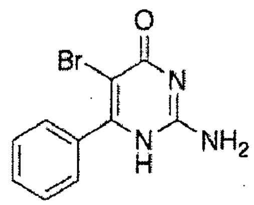

The term "TLR9 ligand" as used herein means a ligand of Toll-like receptor (TLR) 9, and examples include ODN 1826. The TLR9 ligand used in the present invention may be an extract, a product, or a synthetic produce, and it is not limited thereto. In a preferred embodiment of the present invention, the TLR9 ligand is ODN 1826.

ODN 1826 is oligodeoxynucleotide consisting of the following sequence (SEQ NO: 12):

5' - tccatgacgttcctgacgtt - 3'

The term "TLR2/6 ligand" as used herein means a ligand of a heterodimer of Toll-like receptor (TLR) 2 and Toll-like receptor (TLR) 6. Examples include diacylated lipoprotein derived from a cell wall of mycoplasma, and a salt thereof, which may be an extract, a product, or a synthesized product, but are not limited thereto. In a preferred embodiment of the present invention, the TLR2/6 ligand is $Pam_2$ $CSK_4$, MALP-2 and/or FSL-1.

$Pam_2CSK_4$ is represented by the following formula:

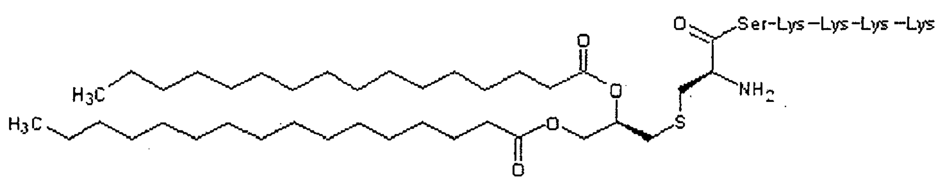

FSL-1 is represented by the following formula:

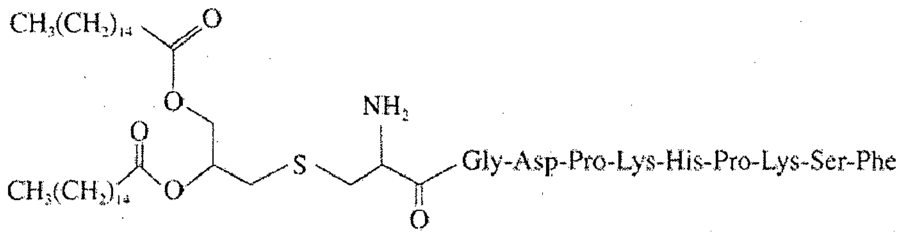

The term "TLR5 ligand" as used herein means a ligand of Toll-like receptor (TLR) 5, and examples include flagellin. The TLR5 ligand used in the present invention may be an extract, a product, or a synthesized product, but is not limited thereto. In a preferred embodiment of the present invention, the TLR5 ligand is flagellin.

The Toll-like receptor (TLR) is a type I transmembrane protein family, which initiates a congenital immune response involved in the specific cytokine, chemokine and growth factor by its in vivo activation. All TLRs can activate a certain intracellular signaling molecule such as nuclear factor κB (NF-κB) or mitogen-activated protein kinase (MAP kinase), but it seems that specific combination of released cytokine and chemokine is inherent in each TLR. TLRs 3, 7, 8 and 9 include a TLR subfamily which is present in an endosome compartment or in a lysosome compartment of immune cells (dendritic cells, monocytes, and the like). Specifically, TLR3 is expressed by a wide range of cells such as dendritic cells and fibroblast; TLR7 is expressed by plasmacytoid dendritic cells and is expressed in a smaller level by monocyte; TLR8 is expressed by monocyte, or monocyte-derived dendritic cells and myeloid dendritic cells; and TLR9 is expressed by plasmacytoid dendritic cells. This subfamily mediates recognition of microorganism nucleic acid (single stranded RNA, double stranded RNA, single stranded DNA, and the like). Agonists of TLR3, TLR7and/or TLR8, or TLR9 stimulate production of various inflammatory cytokines (which include, for example, interleukin-6, interleukin-12, TNF-α, and interferon-γ). Such agonist also promotes the increase in expression of co-stimulators (for example, CD40, CD80, and CD86), major histocompatibility complex molecules, or chemokine receptors. Type I interferon (IFN-α and IFN-β) is also produced by cells when TLR7 and/or TLR8 agonist is activated.

The term "cyclic dinucleotide" as used herein means a molecule which is formed by esterification two nucleotides where the esterification is formed between a OH group in sugar parts of one nucleotide and a phosphoric acid molecule of the other nucleotide to be intermolecularly cyclized, and analogs thereof. Examples include, but are not limited to, cyclic diAMP (c-di-AMP), cyclic diGMP (c-di-GMP), c-dGpGp, c-dGpdGp, c-GpAp, c-GpCp, and c-GpUp. The cyclic dinucleotide activates the dendritic cells or the T cells. Further examples of the cyclic dinucleotide, use thereof as the adjuvant, and production methods thereof are described in JP-A-2007-529531 (Patent Document 5). In a preferred embodiment of the present invention, the cyclic dinucleotide is cyclic diGMP and/or cyclic diAMP. The cyclic diGMP has the formula:

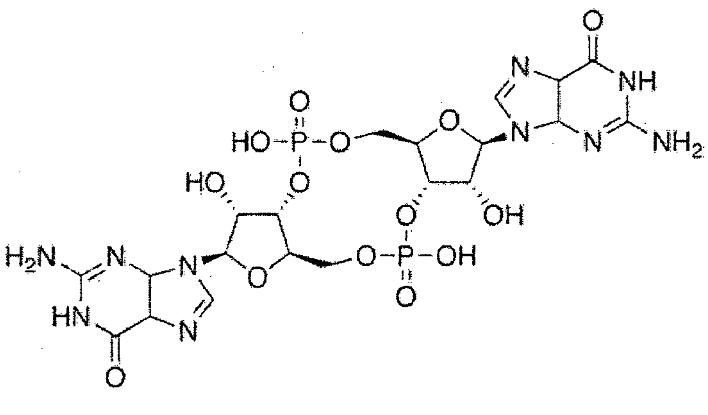

and the synthetic method thereof is described in Kawai et al., Nucleic Acids Research Suppl.3:103-4.

The terms "helper peptide" as used herein means any peptide which activates helper T cells. Examples include tubercle bacillus-derived helper peptide, measles virus-derived helper peptide, hepatitis B virus-derived helper peptide, hepatitis C virus-derived helper peptide, Chlamydia trachomatis-derived helper peptide, tropical malaria Plasmodium sporozoite-derived helper peptide, keyhole limpet haemocyanin-derived helper peptide, tetanus toxin-derived helper peptide, pertussis toxin-derived helper peptide, diphtheria toxin-derived helper peptide, cancer cell-derived helper peptide (for example, IMA-MMP-001 helper peptide, CEA-006 helper peptide, MMP-001 helper peptide, TGFBI-004 helper peptide, HER-2/neu (aa776-790) helper peptide, AE36 helper peptide, AE37 helper peptide, MET-005 helper peptide, and BIR-002 helper peptide), and universal helper analogs (for example, PADRE).

In the present invention, in place of or in combination with the helper peptide described above, a peptide in which all or a part of the amino acids in the helper peptide are modified by substitution, modification or the like (hereinafter, referred to as a "modified helper peptide") can also be used.

Examples of the modified helper peptide include:

(a) a peptide consisting of an amino acid sequence in which one to several, for example, 1, 2, 3, 4 or 5 amino acids are substituted, deleted, or added in the amino acid sequence of the original helper peptide; and
(b) a peptide consisting of an amino acid sequence in which all or a part of amino acids, for example, 1 to several, e.g., 1, 2, 3, 4, 5, 6, 7, 8, 9, 10, 11, 12, 13, 14 or 15 amino acids are modified in the amino acid sequence of the original helper peptide.

Examples of the "modification" in the amino acid which may occur in the modified helper peptide include, but are not limited to, acetylation, alkylation such as methylation, glycosylation, hydroxylation, carboxylation, aldehydation, phosphorylation, sulfonylation, formylation, modification by addition of an aliphatic chain such as myristoylation, palmitoylation or stearoylation, octanoylation, esterification, amidation, deamidation, modification by disulfide bond formation such as cystine modification, glutathione modification or thioglycolic acid modification, glycation, ubiquitination, succinimide formation, glutamylation, and prenylation. The modified helper peptide may include one or more amino acids substituted, deleted or added in combination with one or more amino acids modified.

In a preferable embodiment of the present invention, the helper peptide consists of 10 to 18 amino acids, preferably 12 to 16 amino acids, more preferably 13 to 15 amino acids. In a preferred embodiment of the present invention, the helper peptide is Peptide-25, modified Peptide-25, or PADRE. One example of the modified Peptide-25 is Peptide-25B. The Peptide-25 is a peptide of 15 amino acids consisting of a sequence: Phe Gln Asp Ala Tyr Asn Ala Ala Gly Gly His Asn Ala Val Phe (SEQ NO: 13) and corresponding to amino acid residues 240 to 254 of Ag85B which is one of the major proteins secreted by human tubercle bacillus (Mycobacterium tuberculosis). The Peptide-25B is one example of the modified Peptide-25 obtained by modification of a part of amino acids in the Peptide-25 for the increase in immunostimulation effect, and is a peptide of 15 amino acids consisting of a sequence: Phe Gln Asp Ala Tyr Asn Ala Val His Ala Ala His Ala Val Phe (SEQ NO: 14). PADRE is a peptide of 13 amino acids, consisting of a sequence: D-Ala Lys cyclohexyl-Ala Val Ala Ala Trp Thr Leu Lys Ala Ala D-Ala (which is shown as SEQ NO: 15 in the present application).

The term "immunomodulatory small molecule drug" as used herein means a substance capable of activating or inhibiting immune cells such as T cells, NK cells and macrophage, provided that the substance does not fall into the TLR ligand, the cyclic dinucleotide and the helper peptide described above. Examples of the immunomodulatory small molecule drug include bestatin, pidotimod, levamisole, golotimod, forphenicinol, and their derivatives, and pharmacologically acceptable salts thereof. For example, the pharmacologically acceptable salt of levamisole includes levamisole hydrochloride.

Bestatin is represented by the formula:

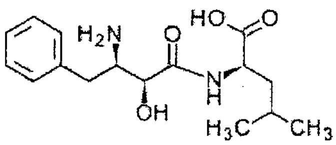

Pidotimod is represented by the formula:

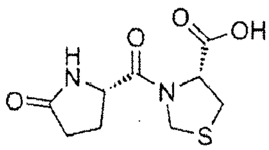

Levamisole hydrochloride is represented by the formula:

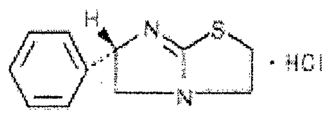

In the present invention, the immunomodulatory small molecule drug is usually a compound having a molecular

weight of less than 1000, preferably less than 500. In a preferred embodiment of the present invention, the immunomodulatory small molecule drug is one or more compounds selected from the group consisting of bestatin, pidotimod and levamisole hydrochloride.

In one embodiment of the present invention, it has been found that a TLR ligand, a cyclic dinucleotide, a helper peptide, or an immunomodulatory small molecule drug is preferably used for increasing the Th1 cell ratio, when a desired antigen is transdermally administered. In one embodiment, accordingly, the cellular immunity induction promoter of the present invention is one or more selected from them. In a further embodiment, this is a first cellular immunity induction promoter. Various methods have been developed as a method for quantitatively measuring the cellular immunity induction, and one or more of them, for example, the ELISPOT method, described in Examples, may be used.

In a preferred embodiment, the cellular immunity induction promoter contained in the vaccine composition for transdermal administration of the present invention is one or more members selected from the group consisting of a TLR ligand and a helper peptide, more preferably one or more selected from the group consisting of TLR7 and/or TLR8 ligand, and a helper peptide.

The term "non-invasive administration" as used herein means an administration without positively providing physical irritation and/or chemical irritation, preferably an administration without positively providing physical irritation (for example, tape stripping or microneedle) to the skin.

The term "mildly-irritating condition" as used herein means a condition under which irritation to be given to the skin is lower than the irritation generally given in order to improve the skin permeability of the antigen contained in conventional vaccines, or a condition under which irritation is not given to the skin at all. In general, physical and/or chemical stimulation is given to the skin before or simultaneously with the transdermal administration of a conventional vaccine composition so that the antigen can penetrate through the skin. In a preferred embodiment of this invention, examples of the mildly-irritating condition includes a condition with low physical irritation and a condition with low chemical irritation. The condition with low physical irritation is, for example, a condition under which a transepidermal water loss (TEWL) ($g/h{\cdot}m^2$) in the model animal for skin irritation evaluation is 50 or less, preferably 45 or less, more preferably 40 or less, further preferably 35 or less, further more preferably 30 or less. Since the TEWL level is about 2 ($g/h{\cdot}M^2$) in the skin untreated, the TEWL level before the administration of the vaccine composition may be 2 ($g/h{\cdot}m^2$) or more. In one embodiment, the condition with low chemical irritation is a condition under which a thymic stromal lymphopoietin (TSLP) level (pg/mg protein) in the skin of the model animal for skin irritation evaluation is 10000 or less, preferably 9000 or less, more preferably 8000 or less, more preferably 7000 or less, more preferably 6000 or less, more preferably 5000 or less, more preferably 4000 or less, more preferably 3000 or less, more preferably 2000 or less, more preferably 1000 or less, more preferably 900 or less, more preferably 800 or less, more preferably 700 or less, more preferably 600 or less, more preferably 500 or less, most preferably 400 or less. Since the TSLP level is about 1 (pg/mg protein) in the skin untreated, the TSLP level at completion of the administration of the vaccine composition is more than 1 (pg/mg protein), preferably more than 2 (pg/mg protein), more preferably more than 3 (pg/mg protein). The thymic stromal lymphopoietin (TSLP) is a cytokine involved in differentiation or recruitment of Th2 cell, and is useful in the present invention as an indicator of a degree of skin irritation (greater TSLP value means stronger skin irritation), or as an indicator of increase in the number of Th2 cells (decrease of the Th1 cell ratio) caused by the skin irritation. Examples of a method for achieving the condition with low physical irritation include administering the vaccine composition onto an intact skin surface of a subject. More specifically, this method includes omitting a pre-treatment such as tape stripping or microneedle puncture on a skin of the subject before the vaccine composition is administrered to the subject. Examples of a method for achieving the condition with low chemical irritation include that the vaccine composition contains an irritative chemical component in an amount less than that effective to irritate a skin, or the vaccine composition does not contain the irritative chemical component. The irritative chemical component includes chemical components irritative to the skin of the subject, such as ethanol and a surfactant. In the administration of the vaccine composition of the present invention to a desired subject, a specific method for achieving the mildly-irritating condition described above is determined using the model animal for skin irritation evaluation, and the method can be utilized on the administration of the composition to the desired subject, for example, human.

The term "cancer" as used herein means abnormal expression of a cancer gene, for example, a cancer with over-expression, for example, hematopoietic tumor or solid cancer. Examples of the cancer gene include survivin gene, GPC3 gene, HER2/neu gene, MAGE3 gene, MAGE A1 gene, MAGE A3/A6 gene, MAGE A4 gene, MAGE12 gene, proteinase-3 gene, AFP gene, CA-125 gene, CD44 gene, CEA gene, c-Kit gene, c-met gene, c-myc gene, L-myc gene, COX2 gene, CyclinD1 gene, Cytokeratin-7 gene, Cytokeratin-19 gene, Cytokeratin-20 gene, E2F1 gene, E2F3 gene, EGFR gene, Gli1 gene, hCG$\beta$ gene, HIF-1-$\alpha$ gene, HnRNP A2/B1 gene, hTERT gene, MDM gene, MDR-1 gene, MMP-2 gene, MMP-9 gene, Muc-1 gene, Muc-4 gene, Muc-7 gene, NSE gene, ProGRP gene, PSA gene, RCAS1 gene, SCC gene, Thymoglobulin gene, VEGF-A gene, and VEGF-A gene. The cancers accompanied with abnormal expression of survivin gene include, but are not limited to, malignant lymphoma, bladder cancer, lung cancer, and colon cancer. The cancers accompanied with abnormal expression of GPC3 gene include, but are not limited to, liver cancer, bile duct cancer, and gastric cancer. The cancers accompanied with abnormal expression of HER2/neu gene include, but are

not limited to, breast cancer, gastric cancer, ovarian cancer, uterine cancer, bladder cancer, non-small-cell lung cancer, and prostate cancer. The cancers accompanied with abnormal expression of MAGE3 gene include, but are not limited to, melanoma, lung cancer, head and neck cancer, bladder cancer, gastric cancer, esophageal cancer, and liver cancer. The cancers accompanied with abnormal expression of proteinase-3 gene include, but are not limited to, acute myelogenous leukemia and pancreatic cancer.

The terms "abnormal expression of a gene" as used herein means that a gene expression level in cells increases or decreases remarkably, for example two times or more, or four times or more. The term "over-expression" means that the abnormal expression relates to the increase in the expression level. The expression level of gene can be easily measured using any method well-known in the art.

The term "subject" as used herein means any animal whose immune response can be induced by the administration of the vaccine composition in a practical stage, and it typically includes mammals including human, for example, mouse, rat, dog, cat, rabbit, horse, cow, sheep, pig, goat, monkey, and chimpanzee. The particularly preferable subject is human.

The term "model animal for immunological evaluation" as used herein means a model animal used in order to evaluate immunity induction property of the vaccine composition, specifically a model animal used in order to evaluate the cellular immunity induction level and the Th1 cell ratio. An animal whose cellular immunity induction caused by the antigen contained in the vaccine composition can be evaluated in view of compatibility between the antigen in the vaccine composition and MHC class 1 molecule of the animal is used as the model animal for immunological evaluation. For example, a vaccine composition comprising HLA-A*24 MHC-restricted class 1 peptide is evaluated with BALB/c mice. A vaccine composition comprising HLA-A*02 MHC-restricted peptide is evaluated with genetically modified mice in which cellular immunity induction caused by HLA-A*02 MHC-restricted peptide can be evaluated. A vaccine composition comprising another type HLA MHC-restricted peptide is evaluated with an animal with which cellular immunity induction caused by the type HLA MHC-restricted peptide can be evaluated. A vaccine composition compriing a protein antigen is evaluated with an animal having MHC compatible with class 1 epitope which is intended to provide cellular immunity induction among class 1 epitopes contained in the amino acid sequence of the protein antigen. When hair is shaved in order to secure a transdermal administration site, an animal is used in the state in which the skin damage caused by the shaving is satisfactorily recovered.

The term "model animal for skin irritation evaluation" as used herein means a model animal used in order to evaluate the transepidermal water loss (TEWL) which is an indicator of the physical irritation on the skin, or the TSLP which is the skin irritation property of the vaccine composition. C57BL/6 mice are used as the model animal for skin irritation evaluation regardless of the kind.of the antigen included in the vaccine composition. When hair is shaved in order to secure a transdermal administration site, an animal is used in the state in which the skin damage caused by the shaving is satisfactorily recovered.

The term "cancer antigen" as used herein means a substance such as protein or peptide capable of specifically expressing tumor cells or cancer cells, and of inducing an immune response.

The term "cancer antigen peptide" as used herein means a partial peptide derived from a cancer antigen protein, which can induce a cellular immune response. The cancer antigen peptide is usually generated by degradation of a cancer antigen protein, which is a cancer gene product, in the cancer cell, and is presented on the surface of the cancer cell by MHC class I molecule. The cancer antigen peptide used in a cancer vaccine formulation may be an endogenous cancer antigen peptide which is obtained by isolation from a cancer cell and purification, or may be a synthetic peptide having the same amino acid sequence as that of the endogenous cancer antigen peptide. In a preferred embodiment of the present invention, for example, an endogenous or synthetic cancer antigen peptide selected from the group consisting of survivin-2B peptide and/or modified survivin-2B peptide, GPC3 peptide and/or modified GPC3 peptide, HER2/neu_A24 peptide and/or modified HER2/neu_A24 peptide, MAGE3_A24 peptide and/or modified MAGE3_A24 peptide, PR1 peptide and/or modified PR1 peptide, HER2/neu_A02 peptide and/or modified HER2/neu_A02 peptide, MAGE3_A02 peptide and/or modified MAGE3_A02 peptide, and MUC1 peptide and/or modified MUC1 peptide may be used for the cellular immunity induction.

The term "virus antigen" as used herein means a substance derived from a virus or a constituent component thereof, or a substance derived therefrom, which can induce a cellular immune response. A viral disease, accordingly, can be treated or prevented by transdermally administering the virus antigen, preferably together with a cellular immunity induction promoter, to a subject. In a preferred embodiment of the present invention, for example, a peptide selected from the group consisting of IPEP87 peptide and/or modified IPEP87 peptide, and HBVenv peptide and/or modified HBVenv peptide can be used as the virus antigen.

The term "viral disease" as used herein means a disease caused by infection and proliferation of virus. Examples include hepatitis A, hepatitis B, hepatitis C, hepatitis D, hepatitis E, cervical cancer, condyloma acuminatum, HIV infection, Chlamydia infection, and herpes simplex.

II. Vaccine composition for Transdermal Administration

In one aspect of the present invention, the vaccine composition for transdermal administration of the present invention exhibits a high cellular immunity inducing effect by controlling the balance of Th1 cells/Th2 cells in a subject so that Th1 cells are in a dominant state due to transdermal administration of various antigens.

The term "pharmaceutical composition for transdermal administration" as used herein means any formulation usually used for the transdermal administration, and it may be, for example, a liquid formulation for external use such as a liniment formulation or a lotion formulation, a spray formulation for external use such as aerosol, an ointment formulation, a plaster formulation, a cream formulation, a gel formulation, or a adhesive skin patch such as a tape preparation or poultice preparation. The classification, definition, characteristics, and production methods thereof are well-known in the art, and see, for example, the Japanese Pharmacopoeia, the 16th edition.

Examples of a base for a liniment formulation include water, ethanol, fatty oil such as hard paraffin, soft paraffin, liquid paraffin, glycerol, paraffin oil, beeswax, and metal soap; mucilage; natural oil (for example: almond oil, corn oil, peanut oil, castor oil, olive oil, and their derivatives (for example, polyoxyl castor oil)); mutton tallow and its derivative, and fatty acid and/or its ester (for example: stearic acid, oleic acid, and isopropyl myristate).

The lotion formulation is a formulation in which an active component is finely uniformly dispersed in an aqueous liquid, and includes a suspension lotion formulation and an emulsion lotion formulation. Examples of a suspending agent include gum arabic, sodium alginate, sodium carboxymethylcellulose, methylcellulose, and bentonite. Examples of an emulsifying agent include sodium laurylsulfate and sorbitan fatty acid ester.

As an ointment base, for example, oils and fats, wax, and a hydrocarbon compound which are generally a hydrophobic base can be used. Specifically, the ointment base includes mineral bases such as yellow petrolatum, white petrolatum, paraffin, liquid paraffin, plastibase and silicone, and animal and vegetable bases such as beeswax and animal and vegetable oils and fats.

Examples of the cream formulation base include water/oil bases such as hydrophilic ointment and vanishing cream; and oil/water bases such as hydrophilic petrolatum, purified lanolin, Aquahole, Eucerin, Neocerin, hydrous lanolin, cold cream, and hydrophilic plastibase.

As the gel bases, for example, carboxyvinyl polymers, gel base, fat-free ointments, polyvinyl pyrrolidone, polyvinyl alcohol, sodium polyacrylate, carboxymethylcellulose, starch, xanthan gum, karaya gum, sodium alginate, methylcellulose, hydroxypropyl cellulose, hydroxypropyl methylcellulose phthalate (HPMCP), cellulose acetate phthalate (CAP), carboxymethylethylcellulose (CMEC), ethylcellulose, hydroxyethylcellulose, hydroxypropyl methylcellulose, a carboxyvinyl polymer, tragacanth, gum arabic, tara gum, tamarind seed gum, psyllium seed gum, agar, gellan gum, glucomannan, locust bean gum, guar gum, carrageenan, dextrin, dextran, amylose, carboxymethylcellulose potassium, carboxymethylcellulose sodium, carboxymethylcellulose calcium, pullulan, chitosan, carboxymethyl starch sodium, Plantago testa, galactomannan, aminoalkyl methacrylate copolymer E, aminoalkyl methacrylate copolymer RS, methacrylic acid copolymer L, methacrylic acid copolymer LD, methacrylic acid copolymer S, a methylacrylate-methacrylic acid-methyl methacrylate copolymer, an ethyl acrylate-methyl methacrylate copolymer, polyvinyl acetal diethyl aminoacetate, casein, alginic acid alkyl ester, gelatin, and polyethylene glycol, which are hydrogel bases, can be used.

Examples of the base for poultice preparation include gelatin, carboxymethylcellulose sodium, methylcellulose, sodium polyacrylate, kaolin, polyvinyl alcohol, polyvinyl pyrrolidone, glycerol, propylene glycol, and water.

For example, the tape preparation comprises an adhesive layer containing an acrylic adhesive, a natural rubber adhesive, a synthetic rubber adhesive (synthesized isoprene rubber, polyisobutylene (PIB), styrene-butadiene rubber, styrene-isoprene-styrene (SIS) rubber), a silicone adhesive, a vinyl ester adhesive, a vinyl ether adhesive, or the like, and a support which supports the adhesive layer. If desired, the tape preparation may further comprise a release liner which does not allow the adhesive layer to be exposed before use and which can be easily released from the adhesive layer in use.

A ratio between an antigen and a cellular immunity induction promoter contained in the pharmaceutical composition of the present invention is not particularly limited. In one embodiment, the pharmaceutical composition of the present invention preferably comprises a desired antigen in an amount of 0.01 to 40% by weight , more preferably 0.1 to 30% by weight, based on the total weight of the composition. In one embodiment, the pharmaceutical composition of the present invention preferably comprises the cellular immunity induction promoter in an amount of 0.001-30% by weight, more preferably 0.01 to 20% by weight, based on the total weight of the composition.

When the vaccine composition for transdermal administration of the present invention is a tape preparation and the tape preparation is a matrix tape preparation, the tape preparation comprises an adhesive layer which comprises an antigen etc. as an active component, and a support which supports the adhesive layer. When the tape preparation is reservoir tape preparation, the tape preparation comprises a reservoir which comprises an antigen etc. as an active component, an adhesive layer, and a support which supports the reservoir and the adhesive layer. The adhesive layer of the tape preparation (hereinafter, referred to as the "tape preparation of the present invention") comprises an antigen and, if desired, the cellular immunity induction promoter. In one embodiment, the adhesive layer of the tape preparation

of the present invention preferably comprises an antigen in an amount of 0.01 to 40% by weight, more preferably 0.1 to 30% by weight, based on the total weight of the adhesive layer. When the adhesive layer of the tape preparation of the present invention comprises a cellular immunity induction promoter, the cellular immunity induction promoter is preferably contained in an amount of 0.001 to 30% by weight, more preferably 0.01 to 20% by weight, based on the total weight of the adhesive layer.

The adhesive forming the adhesive layer of the tape preparation of the present invention is not particularly limited, and examples include acrylic adhesives including an acrylic polymer; rubber adhesives including a rubber elastomer, such as styrene-diene-styrene-block copolymers (for example, a styrene-isoprene-styrene-block copolymer and a styrene-butadiene styrene-block copolymer), polyisoprene, polyisobutylene, a butyl rubber, and polybutadiene; silicone adhesives such as a silicone rubber, a dimethylsiloxane base, and a diphenylsiloxane base; vinyl ether adhesives such as polyvinyl methyl ether, polyvinyl ethyl ether, and polyvinyl isobutyl ether; vinyl ester adhesives such as a vinyl acetate-ethylene copolymer; and polyester adhesives including a carboxylic acid component such as dimethyl terephthalate, dimethyl isophthalate, or dimethyl phthalate, and a polyhydric alcohol such as ethylene glycol. Particularly preferable adhesives are an acrylic adhesive, a rubber adhesive, and a silicone adhesive. The adhesive is preferably contained in the adhesive layer in an amount of 10 to 90% by weight, more preferably 20 to 80% by weight based on the total weight of the adhesive layer.

Examples of the acrylic adhesive include acrylic acid ester adhesives which comprise as a main component a polymer comprising a $C_2$ to $C_{18}$ alkyl ester of (meth) acrylic acid as a first monomer. Example of the alkyl ester of (meth) acrylic acid (first monomer) include alkyl esters of (meth) acrylic acid in which the alkyl group is a linear, branched or cyclic alkyl group having 1 to 18 carbon atoms (for example, methyl, ethyl, propyl, butyl, pentyl, hexyl, cyclohexyl, heptyl, octyl, 2-ethylhexyl, nonyl, decyl, undecyl, dodecyl, and tridecyl). Alkyl esters of (meth)acrylic acid in which the alkyl group is a linear, branched or cyclic alkyl group having 4 to 18 carbon atoms (for example, butyl, pentyl, hexyl, cyclohexyl, heptyl, octyl, 2-ethylhexyl, nonyl, decyl, undecyl, dodecyl, and tridecyl) are preferable. It is further preferable for providing adhesiveness at an ordinary temperature to use a monomer component which reduces a glass transition temperature of a polymer, and thus alkyl esters of (meth) acrylic acid in which the alkyl group is a linear, branched or cyclic alkyl group having 4 to 8 carbon atoms (for example, butyl, pentyl, hexyl, cyclohexyl, heptyl, octyl, and 2-ethylhexyl, preferably butyl, 2-ethylhexyl, and cyclohexyl, particularly preferably 2-ethylhexyl) are more preferable. Specifically, butyl acrylate, 2-ethylhexyl acrylate, 2-ethylhexyl methacrylate, cyclohexyl acrylate, cyclohexyl methacrylate, and the like are more preferable, and especially, 2-ethylhexyl acrylate is most preferable. These alkyl esters of (meth)acrylic acid may be used alone or as a mixture of two or more.

The acrylic adhesive may comprise a second monomer copolymerizable with the alkyl ester of (meth)acrylic acid described above, and examples of the second monomer include monomers having a functional group which can form a cross-linking point when a cross-linking agent is used. The functional group involved in the cross-linking reaction includes a hydroxyl group, a carboxyl group, and a vinyl group, and a hydroxyl group and a carboxyl group are preferable. Specific examples of the monomer (second monomer component) include hydroxyethyl esters of (meth)acrylic acid, hydroxypropyl esters of (meth)acrylic acid, N-hydroxyalkyl (meth)acrylamide, (meth)acrylic acid, itaconic acid, maleic acid, maleic anhydride, mesaconic acid, citraconic acid, and glutaconic acid. Among them, in terms of ease of obtaining, acrylic acid, methacrylic acid, and hydroxyethyl esters of acrylic acid (in particular, 2-hydroxyethyl acrylate) are preferable, and acrylic acid is most preferable. These monomers (second monomer components) may be used alone or as a mixture of two or more.

Further, the acrylic adhesive may comprise, if desired, a third monomer other than the second monomer. Examples of the third monomer (third monomer component) include vinyl esters such as vinyl acetate and vinyl propionate; vinyl ethers such as methyl vinyl ether and ethyl vinyl ether; vinyl amides such as N-vinyl-2-pyrrolidone and N-vinyl caprolactam; alkoxyesters of (meth)acrylic acid such as methoxyethyl ester of (meth) acrylic acid, ethoxyethyl ester of (meth) acrylic acid, and tetrahydrofuryl ester of (meth)acrylic acid; hydroxyl group-containing monomers such as hydroxypropyl (meth)acrylate and $\alpha$-hydroxymethyl acrylate (which does not act as a cross-linking point because it is used as the third monomer component); (meth)acrylic acid derivatives having an amido group such as (meth)acrylamide, dimethyl (meth)acrylamide, N-butyl (meth)acrylamide, and N-methylol (meth)acrylamide; aminoalkyl esters of (meth) acrylic acid such as aminoethyl ester of (meth)acrylic acid, dimethylaminoethyl ester of (meth)acrylic acid, and t-butylaminoethyl ester of (meth)acrylic acid; alkoxy alkylene glycol esters of (meth)acrylic acid such as methoxy ethylene glycol ester of (meth)acrylic acid, methoxy diethylene glycol ester of (meth)acrylic acid, methoxy polyethylene glycol ester of (meth)acrylic acid, and methoxy polypropylene glycol ester of (meth)acrylic acid; (meth)acrylonitrile; monomers having sulfonic acid such as styrenesulfonic acid, allyl sulfonic acid, sulfopropyl (meth)acrylate, (meth)acryloyloxynaphthalenesulfonic acid, and acrylamide methylsufonic acid; and vinyl group-containing monomers such as vinyl piperidone, vinylpyrimidine, vinylpiperazine, vinylpyrrole, vinylimidazole, vinyloxazole, and vinylmorpholine. Among them, vinyl esters and vinyl amides are preferable, and vinyl acetate is preferable for vinyl esters and N-vinyl-2-pyrrolidone is preferable for vinyl amides. These monomers (third monomer components) may be used alone or as a mixture of two or more.

When the acrylic adhesive is a copolymer of alkyl ester of (meth)acrylic acid (first monomer component) with

vinyl monomer having a functional group capable of being involved in the cross-linking reaction (second monomer component), it is preferable to blend and copolymerize the alkyl ester of (meth)acrylic acid with the vinyl monomer capable of being involved in the cross-linking reaction in a weight ratio of the alkyl ester of (meth)acrylic acid : the vinyl monomer having a functional group capable of being involved in the cross-linking reaction = 99 to 85 : 1 to 15, more preferably in a weight ratio of 99 to 90 : 1 to 10.

When the acrylic adhesive is a copolymer of alkyl ester of (meth) acrylic acid (first monomer component), vinyl monomer having a functional group capable of being involved in the cross-linking reaction (second monomer component) and the monomer other than the above (third monomer component), it is preferable to blend and copolymerize the alkyl ester of (meth) acrylic acid, the vinyl monomer having a functional group capable of being involved in the cross-linking reaction and the monomer other than the above in a weight ration of the alkyl ester of (meth)acrylic acid : the the vinyl monomer having a functional group capable of being involved in the cross-linking reaction : the monomer other than the above = 40 to 94 : 1 to 15 : 5 to 50, more preferable in a weight ratio of 50 to 89 : 1 to 10 : 10 to 40.

The polymerization reaction may be performed in a known method and is not particularly limited. Examples include a method in which the monomers described above are allowed to be reacted in a solvent (for example, ethyl acetate) at 50 to 70°C for 5 to 48 hours by addition of a polymerization initiator (for example, benzoyl peroxide and azobisisobutyronitrile).

In the present invention, examples of the particularly preferable acrylic adhesive include copolymers of 2-ethylhexyl ester of acrylic acid/acrylic acid/N-vinyl-2-pyrrolidone, copolymers of 2-ethylhexyl ester of acrylic acid/N-(2-hydroxyethyl)acrylamide/N-vinyl-2-pyrrolidone, copolymers of 2-ethylhexyl ester of acrylic acid/2-hydroxyethyl ester of acrylic acid/vinyl acetate, and copolymers of 2-ethylhexyl ester of acrylic acid/acrylic acid; and more preferable examples include copolymers of 2-ethylhexyl ester of acrylic acid/acrylic acid/N-vinyl-2-pyrrolidone.

If desired, the acrylic adhesive may be subjected to a physical cross-linking treatment by exposure of radiation such as ultraviolet irradiation or electron beam irradiation; or a chemical cross-linking treatment using various cross-linking agents, for example, an isocyanate compound such as trifunctionalisocyanate, an organic peroxide, an organic metal salt, a metal alcoholate, a metal chelate compound, or a polyfunctional compound (polyfunctional external cross-linking agent, and polyfunctional monomer for internal cross-linking, such as diacrylate or dimethacrylate).

Examples of the rubber adhesive include rubber adhesives comprising as a rubber elastomer such as a polyisobutylene-polybutene elastomer, a styrene-diene-styrene block copolymer, a styrene-butadiene elastomer, a nitrile elastomer, a chloroprene elastomer, a vinylpyridine elastomer, a polyisobutylene elastomer, a butyl elastomer, and an isoprene-isobutylene elastomer. Among them, in terms of the solubility in the peptide and the cellular immunity induction promoter therefor and the skin adhesiveness, polyisobutylene (PIB), styrene-diene-styrene block copolymers (for example, styrene-butadiene-styrene block copolymer (SBS) and styrene-isoprene-styrene block copolymer (SIS)) are preferably used. They may be used as a mixture.

In order to obtain an appropriate adhesion force and drug solubility, as the rubber adhesive, a mixture of rubber elastomers which have the same component or a different component and have a different average molecular weight can be used. For example, in a case where a polyisobutylene is explained as an example, a mixture of a high molecular weight polyisobutylene having an average molecular weight of 150, 000 to 5,500,000, and a middle molecular weight polyisobutylene having an average molecular weight 10,000 to 150,000 and/or a low molecular weight poly isobutylene having an average molecular weight of 500 to 4,000 is preferable. Here, it is preferable to blend the high molecular weight, middle molecular weight and low molecular weight polyisobutylenes in a weight ratio of the high molecular weight polyisobutylene : the middle molecular weight polyisobutylene : the low molecular weight polyisobutylene = 10 to 80, preferably 20 to 70 : 0 to 9.0, preferably 10 to 80 : 0 to 80, preferably 10 to 60.

The "average molecular weight" in the present invention means a viscosity average molecular weight calculated from the Flory's viscosity formula, and the viscosity average molecular weight is determined by using a Staudinger index ($J_O$) from the following formulae, the value $J_O$ being calculated from a flow time of a capillary 1 of a Ubbelohde viscometer at 20°C using the Schulz-Blaschke formula.

(Formula)

$$J_0 = \eta_{sp}/c(1 + 0.31\eta_{sp}) \quad \text{(Schulz-Blaschke equation)}$$

$$\eta_{sp} = t/t_0 - 1$$

t: Flow time of solution (according to Hagenbach-couette correction formula)
$t_0$: Flow time of solvent (according to Hagenbach-couette correction formula)
c: Concentration of solution ($g/cm^3$)

$J_0 = 3.06 \times 10^{-2}\ \overline{Mv}^{0.65}$

$\overline{Mv}$: Viscosity average molecular weight

The rubber adhesive may comprise, for example, a tackifier such as a rosin resin, a polyterpene resin, a coumarone-indene resin, a petroleum resin, a terpene-phenol resin, a xylene resin, or a saturated alicyclic hydrocarbon resin blended therewith, in order to provide appropriate adhesiveness . One or more of the tackifiers may be blended in an amount of 50% by weight or less, preferably 5 to 40% by weight, based on the total weight of the rubber adhesive.

Examples of the silicone adhesive include silicone adhesives such as a polyorganosiloxane adhesive, polydimethylsiloxane adhesive, and a polydimethyldiphenyl-siloxane adhesive. Among them, silicone adhesives commercially available from Dow Corning Corporation are preferably used.

The support which supports the adhesive layer is not particularly limited, but supports which are substantially impermeable to the peptide or the cellular immunity induction promoter, i.e., supports, where the peptide, the cellular immunity induction promoter, the additives and the like included in the adhesive layer do not pass through and are not lost from the back surface, and the reduction in the contents thereof is not caused, are preferable.

As the support, for example, a mono-layered film or a laminated film of polyester, polyamide, polyvinylidene chloride, polyethylene, polypropylene, polyvinyl chloride, an ethylene-ethyl acrylate copolymer, polytetrafluoroethylene, an ionomer resin, a metal foil, and the like may be used. Among them, the support is preferably a laminated film of a non-porous plastic film and a porous film which are formed of the material described above in order to improve attachment property (anchoring property) between the support and the adhesive layer. In this case, it is desirable that the adhesive layer is formed on the side of the porous film. As such a porous film, a film which improves the anchoring property to the adhesive layer is adopted, and specific examples thereof include paper, a woven fabric, a non-woven fabric, a knitted fabric, and a sheet subjected to a mechanical perforation. Among them, paper, a woven fabric and a non-woven fabric are particularly preferable in terms of handleability. A porous film having a thickness in a range from 1 to 200 μm is adopted in term of the improvement in anchoring property, the softness of the tape preparation, and attachment operability. When a woven fabric or a non-woven fabric is used as the porous film, the basis weight thereof is preferably from 5 to 30 g/$m^2$, more preferably from 6 to 15 g/$m^2$.

The most preferable support is a laminated film of a polyester film (preferably polyethylene terephthalate film) having a thickness of 1.5 to 6 μm, and a polyester (preferably polyethylene terephthalate) non-woven fabric having a basis weight of 6 to 15 g/$m^2$.

Laminating a release liner on the adhesive surface is desirable for the tape preparation of the present invention in order to protect the adhesive surface of the adhesive layer beforeuse.. The release liner is not particularly limited so long as a release treatment is performed and a sufficient light peel force is secured. For example, a film such as polyester, polyvinyl chloride, polyvinylidene chloride and polyethylene terephthalate; or paper such as wood free paper or glassine paper; or a laminated film of wood free paper or glassine paper and polyolefin, whose surface in contact with the adhesive layer is subjected to a release treatment by coating with a silicone resin or a fluorine-containing resin is used for the release liner. The release liner preferably has a thickness of 10 to 200 μm, more preferably 25 to 100 μm. The release liner is preferably formed of a polyester (particularly polyethylene terephthalate) resin in terms of barrier property, price, and the like. In this case, one having a thickness of about 25 to 100 μm is further preferably in terms of the handling.

The pharmaceutical composition of the present invention, if necessary, may comprise an additive. The additive can be selected, for example, from an isotonizing agent, an antiseptic/antimicrobial agent, an antioxidant, a solubilizer, a solubilizing aid, a suspending agent, filler, a pH-controlling agent, a stabilizer, absorption promoter, a releasing speed controller, a coloring agent, a plasticizer, a cross-linking agent, an adhesive, and a mixture of two or more thereof, according to the compatibility with the main component of the base, the antigen, and the cellular immunity induction promoter, the desired dosage regimen, and the like. When the pharmaceutical composition of the present invention is a tape preparation, the tape preparation may comprise a skin permeability enhancer as the additive.

The terms "skin permeability enhancer" as used herein means any substance which can more improve the efficiency of the antigen transdermally administered into the skin, as compared to the efficiency when the antigen is administered without the substance. The skin permeability enhancer is not particularly limited so long as it is liquid at room temperature (25°C), i.e., it has fluidity, or when it is used as a mixture of two or more, the final mixture is liquid at room temperature (25°C) and has an effect of promoting absorption. Such an organic liquid component is preferably a hydrophobic liquid component in terms of the compatibility with the adhesive layer.

Examples of the skin permeability enhancer include higher alcohols such as oleyl alcohol and octyl decanol; polyhydric alcohols such as glycerol, ethylene glycol and polypropylene glycol; higher fatty acids such as oleic acid and caprylic acid; esters of fatty acid such as isopropyl myristate, isopropyl palmitate and ethyl oleate; esters of polybasic acid such as diethyl sebacate and diisopropyl adipate; fatty acid esters of polyhydric alcohol such as diglyceryl triisostearate, sorbitan monooleate, propylene glycol dicaprylate, polyethylene glycol monolaurate and polyoxyethylene sorbitol tetraoleate; polyoxyethylene alkyl ethers such as polyoxyethylene lauryl ether; hydrocarbons such as squalane and liquid paraffin; vegetable oil such as olive oil and castor oil; silicone oil; pyrrolidones such as N-methyl pyrrolidone and

N-dodecyl pyrrolidone; and sulfoxides such as decyl methyl sulfoxide. They may be used alone or as a mixture of two or more.

When the rubber or acrylic adhesive is used, a second skin permeability enhancer may be used. Specific examples of the second skin permeability enhancer include, but are not limited to, polyvinyl pyrrolidone, crospovidone, polypropylene glycol, polyvinyl alcohol, a carboxyvinyl polymer, hydroxypropyl cellulose, and mixtures thereof. In a preferred embodiment, the second skin permeability enhancer used for the present invention is polyvinyl pyrrolidone, crospovidone, and/or polypropylene glycol.

As the skin permeability enhancer, a higher alcohol, more specifically a higher alcohol having 8 to 18 (preferably 8 to 14) carbon atoms; and a fatty acid ester, more specifically a fatty acid ester of a fatty acid having 8 to 18 (preferably 12 to 16) carbon atoms and a monohydric alcohol having 1 to 18 carbon atoms, a fatty acid ester of polyhydric alcohol, particularly fatty acid ester, particularly isopropyl myristate, isopropyl palmitate, or diethyl sebacate is preferably used in terms of the skin permeability enhancement of the antigen peptide. The amount of the skin permeability enhancer is preferably from 0.1% by weight to 70% by weight, more preferably from 1% by weight to 65% by weight, more preferably from 5% by weight to 60% by weight, based on the total weight of the adhesive layer. When the amount of the skin permeability enhancer is 0.1% by weight or more, a high transdermal absorption promoting effect can be obtained. When it is 70% by weight or less, while the reduction in the adhesion force of the adhesive layer as a whole and the reduction in cohesion force are suppressed, a high transdermal absorption property can be advantageously obtained.

The pharmaceutical composition of the present invention is preferably administered to a subject in a mildly-irritating condition. The administration in a mildly-irritating condition can be attained, for example, by (i) the administration of the pharmaceutical composition of the present invention to the subject in an administration condition in which a transepidermal water loss (TEWL) ($g/h \cdot m^2$) is 50 or less when a model animal for skin irritation evaluation is evaluated; (ii) the administration of the pharmaceutical composition to the subject so that a cutaneous TSLP level (pg/mg protein) is 10000 or less when a model animal for skin irritation evaluation is evaluated, or the like.

The pharmaceutical composition of the present invention can further comprise a pharmacologically acceptable acid or a pharmacologically acceptable salt thereof to improve cellular immunity induction promoting effect.

The "pharmacologically acceptable acid" as used herein, which can be contained in the pharmaceutical composition of the present invention as the second cellular immunity induction promoter, means an acid which does not provide a harmful effect to a subject to be administered, and does not extinguish the pharmacological activity in the component in the pharmaceutical composition. In a preferred embodiment of the present invention, the pharmacologically acceptable acid is an organic acid; more preferably an organic compound containing carboxyl group or an organic compound containing sulfonate group; more preferably a saturated or unsaturated linear or branched fatty acid having a saturated linear portion having 8 to 20 carbon atoms, lactic acid, malic acid, salicylic acid, maleic acid, citric acid, or an organic compound containing sulfonate group; more preferably a saturated or unsaturated linear or branched fatty acid having a saturated linear portion having 8 to 16 carbon atoms, lactic acid, malic acid, salicylic acid, maleic acid, citric acid, or an organic compound containing sulfonate group; more preferably a fatty acid selected from the group consisting of decanoic acid, lauric acid, myristic acid, isostearic acid, palmitic acid, stearic acid and oleic acid, or lactic acid, salicylic acid, citric acid or methanesulfonic acid.

The "pharmacologically acceptable salt" as used herein, which can be contained in the pharmaceutical composition of the present invention, means a salt which does not provide a harmful effect to a subject to be administered, and does not extinguish the pharmacological activity in the component in the pharmaceutical composition. The salts include, but are not limited to, inorganic acid salts (for example, hydrochloride and phosphate), organic acid salts (for example, acetate, phthalate, and TFA salt), metal salts (alkali metal salts (for example, sodium salt and potassium salt), alkaline earth salts (for example, calcium salt and magnesium salt), aluminum salts, and the like), and amine salts (triethyl amine salt, benzyl amine salt, diethanol amine salt, t-butyl amine salt, dicyclohexyl amine salt, arginine salt, dimethyl ammonium salt, ammonium salt, and the like).

The therapeutically effective amount of the antigen can widely vary depending on the severity of a disease, the age and relative health of a subject, and other known factors, and in general, a daily dose of about 0.1 μg to 1 g/kg body weight can provide a satisfactory result. The cellular immunity induction promoter is administered simultaneously or sequentially when the antigen is administered, preferably, simultaneously. The effective amount of the cellular immunity induction promoter can widely vary depending on the specific cellular immunity induction promoter to be used, and the presence or absence of another cellular immunity induction promoter, and an amount of 0.01 μg to 1 g/kg body weight can provide a satisfactory result. A daily dose can be administered once, and it may be divided into two or more, for example, two, three, four or five aliquots, and administered. The continuous administration time per administration is appropriately selected between one minute and 7 days. The interval between administrations is appropriately selected depending on the state of a patient, the severity of a disease, and whether the purpose is therapeutic or preventive, from every day to once a year (for example, once a day, once every two days, once every three days, once a week, once every two weeks, once a month, once every three months, once every six months, or once a year) or a longer time. In general, for the therapeutic purpose of a patient actually suffering from a severe disease, the antigen is more

frequently administered at a higher dose, and for the preventive purpose of a patient suffering from no disease, the antigen is lower frequently administered at a lower dose.

As used herein, the "physical irritation" means any physical irritation which damages corneum, including scratch and abrasion. Examples of the physical irradiation include a tape stripping operation in which the corneum is removed with an adhesive tape, an operation giving injury on skin with a cutter, and an operation using a microneedle in which a hole is made in the corneum with a minute needle.

The "transepidermal water loss" means an amount (g) of water transpired from 1 $m^2$ of corneum per hour. The transepidermal water loss can be easily measured in a short time by using a water transpiration measuring device, and is generally widely used as an indicator for evaluation of a degree of skin injury. For the present invention, the transepidermal water loss may be used as the indicator of the physical irritation level.

TSLP (Thymic stromal lymphopoietin) is one of IL-7-like cytokines produced from keratinocyte of skin, thymus gland, or mucosal epithelial cells, and it is known to be involved in maturation of dendritic cells and T cell differentiation. For the present invention, the TSLP level can be used as the indicator of the level of chemical irritation derived from the drug or the additive.

The present invention will be more particularly and specifically described below with reference to Examples. The present invention, however, is not limited to the scope of Examples.

EXAMPLES

Liquid Formulation for External Use

Each liquid formulation for external use having each composition shown in Tables 1 to 11 was produced. An antigen peptide in an amount of parts by weight set forth in Tables, 3 parts by weight of a cellular immunity induction promoter other than a helper peptide, 0.3 parts by weight of the helper peptide, and 20 parts by weight of DMSO were blended, a base material was added thereto so that the total amount was 100 parts by weight, and the resultant was mixed to provide a liquid formulation for external use. With respect to each of the liquid formulations for external use of Test Examples, where the amount blended was specified in Tables, the amount of each component blended was as shown in the Tables. As the base material, one prepared by mixing and blending propylene glycol (PG) and oleyl alcohol (OA) in a weight ratio of 98 : 2, 90 : 10, 87.5 : 12.5 or 85 : 15 was used.

Imiquimod was purchased from Tokyo Chemical Industry Co. , Ltd. With respect to GPC3 peptide, survivin-2B peptide, HER2/neu_A24 peptide, MAGE3_A24 peptide, IPEP87 peptide, HER2/neu E75 peptide, PR1 peptide, HER2/neu_A02 peptide, MAGE3_A02 peptide, HBVenv peptide, MUC1 peptide, Peptide-25 and Peptide-25B, chemically synthesized and HPLC-purified products were used.

A composite base material, in which a cellulose non-woven fabric (area: 0.8 $cm^2$) was bonded to the central part of an adhesive tape for fixing, was prepared. The composite base material in which the non-woven part thereof was impregnated with 67 $\mu$L of the prepared liquid formulation for external use was used as an administration sample for immunity tests.

Mouse Immunity Test 1 (Liquid Formulation for External Use)

With respect to each of the liquid formulations for external use, a mouse immunity test was performed using a model animal for immunological evaluation. An immunity induction level was evaluated in accordance with the ELISPOT method. Specifically, a back of a mouse was shaved followed by a period for recovering the skin damage caused by the shaving, and then, the sample was administered to the back skin of the mouse in a predetermined time and removed therefrom. The mouse was kept for predetermined days, and a level of antigen-specifically cellular immunity induction was evaluated. After the predetermined days passed from the administration, the spleen thereof was removed, and a suspension of splenocytes was prepared. Splenocytes ($3 \times 10^6$ cells/well) and an antigen peptide (100 $\mu$M) (when a protein is used as an antigen, 100 $\mu$g/mL of the antigen protein) were added to wells of an ELISPOT plate to which antimouse IFN-$\gamma$ antibodies were immobilized, together with a culture medium, which was co-cultured in culture conditions of 37°C and 5% $CO_2$ for 20 hours, and the number of spots of IFN-$\gamma$ productive cells (the number of spots/$3 \times 10^6$ cells) was evaluated in accordance with the ELISPOT method. The dosage of each of the liquid formulations for external use was, as described above, was 67 $\mu$L, the number of administrations thereof was (24 hours/week) $\times$ once, and the spleen was removed after 6 days from the administration.

Furthermore, a percentage (Th1 cell ratio) of the number of Th1 cells to the total number of Th1 cells and Th2 cells in a subject was measured in accordance with the following method.

In Test Example 6, the administration was performed on a skin pre-treated by ten times of tape stripping (TS) with DUNPLON tape (No. 375 manufactured by Nitto Denko CS System

Corporation).

In addition, with respect to a part of the liquid formulations for external use, the cutaneous TSLP level of the mouse after the administration and the transepidermal water loss of the mouse before the administration were also measured in accordance with a method described below.

(Measurement of Th1 Cell Ratio)

When a sample to be used for immunization contains a helper peptide or a protein antigen, the ratio of Th1 cells was determined according to the following procedure.

Using the suspension of splenocytes obtained in the mouse immunity test, splenocytes ($1 \times 10^6$ cells/well) and the helper peptide (100 μM) used for the immunity were added to wells of an ELISPOT plate to which antimouse IFN-γ antibodies were immobilized, and wells of an ELISPOT plate to which antimouse IL-4 antibodies were immobilized, together with a culture medium, which was co-cultured in culture conditions of 37°C and 5% $CO_2$ for 20 hours, and the number of spots of IFN-γ productive cells (the number of spots/$1 \times 10^6$ cells) and the number of spots of IL-4 productive cells (the number of spots/$1 \times 10^6$ cells) were evaluated in accordance with the ELISPOT method. In such a test in which immunity was provided by a vaccine including a protein antigen such as OVA protein among the evaluation methods described above, co-cultivation was performed using a protein antigen (100 μg/mL) instead of the helper peptide (100 μM). The Th1 cell ratio (%) was calculated from the calculation formula: Th1 cell ratio (%) = (the number of Th1 cells × 100) / (the number of Th1 cells + the number of Th2 cells) wherein the number of Th1 cells is the number of spots of the IFN-γ productive cells, and the number of Th2 cells is the number of spots of the IL-4 productive cells.

(Method for Measuring TSLP Level)

A TSLP level was evaluated using C57BL/6 mice, which were model animals for skin irritation evaluation. The formulation was administered to model animals for skin irritation evaluation in the same administration conditions as those to the model animals for immunological evaluation, the back skin of a mouse was removed at the end of the administration of the formulation, and the skin was pulverized in an extracting solvent (PBS solution including a protease inhibitor (Protease Inhibitor Cocktail for general use, manufactured by SIGMA-ALDRICH) and 10 μM of indomethacin (manufactured by Wako Pure Chemical Industries, Ltd.)) using a homogenizer (Physcotron manufactured by Microtec Co., Ltd. ). The pulverized skin was centrifuged at 4°C in 9000 g for 10 minutes, and then the supernatant thereof was recovered. The amount of TSLP in the supernatant was measured by using ELISA (Mouse TSLP Quantikine ELISA Kit manufactured by R&D Systems). The total amount of protein in the supernatant was measured by the BCA method (Pierce BCA Protein Assay Kit, manufactured by Thermo SCIENTIFIC), and standardization was performed by dividing the amount of TSLP by the total amount of protein.

(Measurement of Transepidermal Water Loss)

Using C57BL/6 mice, which were model animals for skin irritation evaluation, the transepidermal water loss of skin before the administration of the formulation was evaluated. Using a portable, closed chamber type water transpiration measuring device (VAPO SCAN AS-VT100RS manufactured by Asahibiomed), a measurement was performed by bringing the mouse skin into contact with the device for about 5 to 15 seconds. The value measured after 10 minutes from the pre-treatment to the mouse skin was defined as a transepidermal water loss (TEWL) ($g/h \cdot m^2$).

The results of the immunity tests, and the measurement results of the Th1 cell ratio, the TSLP level, and the transepidermal water loss are shown in Tables 1 to 11 below together with the mice used. The "genetically modified mice" in Tables are genetically modified mice from which the cellular immunity induction owing to HLA-A* 0201 MHC-restricted peptide can be evaluated. For comparison, the results obtained from immunity caused by injection formulations described below

(Reference Examples 1 to 8) were described at the end of each Table.

Table 1

| | Composition | | | | Chemical irritation | TSLP (pg/mg protein) | Physical irritation | TEWL ($g/h \cdot m^2$) | Mouse for immunological evaluation | Results of immunization (ELISPOT average number of spots) | Ratio of Th1 cells (%) |
|---|---|---|---|---|---|---|---|---|---|---|---|
| | Base | Antigen peptide | Cellular immunity induction promoter | | | | | | | | |
| Test example 1 | PG/OA[98/2] | Survivin 2B(10) | IMQ(3) | PEPB (0.3) | None | 186 | None | 10 | BALB/c | 1477 | 40 |
| Reference Example 1 | Saline | Survivin 2B (0.125) | Montanide ISA51VG (50) | | None | | None | | BALB/c | 313 | Unevaluable |

PG/OA: Mixture of propylene glycol and oleyl alcohol (both are from Wako Pure Chemical Industries, Ltd.). Numbers in [ ] show a weight ratio of PG to OA. Numbers in () are a blending ratio of each component (parts by weight). (The same applies to the following Tables.)
IMQ: Imiquimod (TLR7 and/or TLR8 ligand)
PEPB: Peptide-25B (SEQ NO: 14)

Table 2

| | Composition | | | | Chemical irritation | TSLP (pg/mg protein) | Physical irritation | TEWL (g/h·m$^2$) | Mouse for immunological evaluation | Results of immunization (ELISPOT average number of spots) | Ratio of Th1 cells (%) |
|---|---|---|---|---|---|---|---|---|---|---|---|
| | Base | Antigen peptide | Cellular immunity induction promoter | | | | | | | | |
| Test example 2 | PG/OA[98/2] | GPC3(5) | IMQ(3) | PEPB (0.3) | None | 214 | None | 10 | BALB/c | 96 | 14 |
| Reference Example 2 | Saline | GPC3 (0.125) | Montanide ISA51VG (50) | | None | | None | | BALB/c | 10 | Unevaluable |

Table 3

| | Composition | | | | Chemical irritation | TSLP (pg/mg protein) | Physical irritation | TEWL (g/h·m$^2$) | Mouse for immunological evaluation | Results of immunization (ELISPO average number of spots) | Ratio of Th1 cells (%) |
|---|---|---|---|---|---|---|---|---|---|---|---|
| | Base | Antigen peptide | Cellular immunity induction promoter | | | | | | | | |
| Test example 3 | PG/OA[85/15] | HER2/neu_A29 (5) | IMQ(3) | PEPB (0.3) | Given (Increase in OA ratio) | 12300 | None | 10 | BALB/c | 0 | |
| Test example 4 | PG/OA [87.5/12.5] | HER2/neu_A24 (5) | IMQ(3) | PEPB (0.3) | Given (Increase in OA ratio) | 4500 | None | 10 | BALB/c | 845 | |
| Test example 5 | PG/OA[90/10] | HER2/neu_A24 (5) | IMQ(3) | PEPB (0.3) | Given (Increase in OA ratio) | 315 | None | 10 | BALB/c | 1779 | 12 |
| Test example 6 | PG/OA[98/2] | HER2/neu_A24 (5) | IMQ(3) | PEPB (0.3) | None | 202 | TS 10 times | 58 | BALB/c | 186 | 6 |
| Test example 7 | PG/OA[98/2] | HER2/neu_A24 (5) | IMQ(3) | PEPB (0.3) | None | 188 | None | 10 | BALB/c | 2181 | 16 |
| Reference Example 3 | Saline | HER2/neu_A24 (0.125) | Montanide ISA51VG (50) | | None | | None | | BALB/c | 234 | Unevaluable |
| TS: Tape stripping | | | | | | | | | | | |

Table 4

| | Composition | | | | Chemical irritation | TSLP (pg/mg protein) | Physical irritation | TEWL ($g/h \cdot m^2$) | Mouse for immunological evaluation | Results of immunization (ELISPOT average number of spots) | Ratio of Th1 cells (%) |
|---|---|---|---|---|---|---|---|---|---|---|---|
| | Base | Antigen peptide | Cellular immunity induction promoter | | | | | | | | |
| Test example 8 | PG/OA[98/2] | MAGE3_A24 (10) | IMQ(3) | PEPB (0.3) | None | 216 | None | 10 | BALB/c | 1445 | 45 |
| Reference Example 4 | Saline | MAGE3_A24 (0.125) | Montanide ISA51VG (50) | | None | | None | | BALB/c | 56 | Unevaluable |

Table 5

| | Composition | | | | Chemical irritation | TSLP (pg/mg protein) | Physical irritation | TEWL (g/h·m$^2$) | Mouse for immunological evaluation | Results of immunization (ELISPOT average number of spots) | Ratio of Th1 cells (%) |
|---|---|---|---|---|---|---|---|---|---|---|---|
| | Base | Antigen peptide | Cellular immunity induction promoter | | | | | | | | |
| Test example 9 | PG/OA[98/2] | IPEP87 (10) | IMQ(3) | PEP (0.3) | None | 188 | None | 10 | Genetically modified mice | 1260 | 75 |
| PEP: Peptide-25(SEQ NO: 13) | | | | | | | | | | | |

Table 6

| | Composition | | | | Chemical irritation | TSLP (pg/mg protein) | Physical irritation | TEWL (g/h·m$^2$) | Mouse for immunological evaluation | Results of immunization (ELISPOT average number of spots) | Ratio of Th1 cells (%) |
|---|---|---|---|---|---|---|---|---|---|---|---|
| | Base | Antigen peptide | Cellular immunity induction promoter | | | | | | | | |
| Test example 10 | PG/OA[98/2] | HER2/neu E75(10) | IMQ(3) | PEP (0.3) | None | 224 | None | 10 | Genetically modified mice | 882 | 63 |
| Reference Example 5 | Saline | HER2/neu E75 (0.125) | Montanide ISA51VG (50) | | None | | None | | Genetically modified mice | 110 | Unevaluable |

Table 7

| | Composition | | | | Chemical irritation | TSLP (pg/mg protein) | Physical irritation | TEWL (g/h·m$^2$) | Mouse for immunological evaluation | Results of immunization (ELISPOT average number of spots) | Ratio of Th1 cells (%) |
|---|---|---|---|---|---|---|---|---|---|---|---|
| | Base | Antigen peptide | Cellular immunity induction promoter | | | | | | | | |
| Test example 11 | PG/OA[98/2] | PR1 (5) | IMQ(3) | PEP (0.3) | None | 186 | None | 10 | Genetically modified mice | 1710 | 65 |
| Reference Example 6 | Saline | PR1 (0.125) | Montanide ISA51VG (50) | | None | | None | | Genetically modified mice | 144 | Unevaluable |

Table 8

| | Composition | | | | Chemical irritation | TSLP (pg/mg protein) | Physical irritation | TEWL ($g/h \cdot m^2$) | Mouse for immunological evaluation | Results of immunization (ELISPOT average number of spots) | Ratio of Th1 cells (%) |
|---|---|---|---|---|---|---|---|---|---|---|---|
| | Base | Antigen peptide | Cellular immunity induction promoter | | | | | | | | |
| Test example 12 | PG/OA[98/2) | MUC1 (10) | IMQ(3) | PEP (0.3) | None | 193 | None | 10 | Genetically modified mice | 92 | 51 |

Table 9

| | Composition | | | | Chemical irritation | TSLP (pg/mg protein) | Physical irritation | TEWL ($g/h \cdot m^2$) | Mouse for immunological evaluation | Results of immunization (ELISPOT average number of spots) | Ratio of Th1 cells (%) |
|---|---|---|---|---|---|---|---|---|---|---|---|
| | Base | Antigen peptide | Cellular immunity induction promoter | | | | | | | | |
| Test example 13 | PG/OA[98/2] | HER2/neu_ A02(5) | IMQ(3) | PEP (0.3) | None | 183 | None | 10 | Genetically modified mice | 540 | 58 |
| Reference Example 7 | Saline | HER2/neu_ A02 (0.125) | Montanide ISA51VG (50) | | None | | None | | Genetically modified mice | 93 | Unevaluable |

Table 10

| | Composition | | | | Chemical irritation | TSLP (pg/mg protein) | Physical irritation | TEWL ($g/h \cdot m^2$) | Mouse for immunological evaluation | Results of immunization (ELISPOT average number of spots) | Ratio of Th1 cells (%) |
|---|---|---|---|---|---|---|---|---|---|---|---|
| | Base | Antigen peptide | Cellular immunity induction promoter | | | | | | | | |
| Test example 14 | PG/OA[98/2] | MAGE3_A02 (10) | IMQ(3) | PEP (0.3) | None | 151 | None | 10 | Genetically modified mice | 916 | 65 |

Table 11

| | Composition | | | | Chemical irritation | TSLP (pg/mg protein) | Physical irritation | TEWL (g/h·m$^2$) | Mouse for immunological evaluation | Results of immunization (ELISPOT average number of spots) | Ratio of Th1 cells (%) |
|---|---|---|---|---|---|---|---|---|---|---|---|
| | Base | Antigen peptide | Cellular immunity induction promoter | | | | | | | | |
| Test example 15 | PG/OA[98/2] | HBVenv (10) | IMQ(3) | PEP (0.3) | None | 132 | None | 10 | Genetically modified mice | 119 | 51 |

Tape preparation

Adhesives (PIB rubber adhesive and acrylic adhesive) used in the tape preparation were prepared as follows:

(Preparation of PIB Rubber Adhesive)

In toluene were dissolved 24 parts by weight of polyisobutylene (Oppanol B200 manufactured by BASF), 36 parts by weight of polyisobutylene (Oppanol B12 manufactured by BASF), and 40 parts by weight of an alicyclic petroleum resin (Alcon P-100 manufactured by Arakawa Chemical Industries, Ltd.) to provide a solution of a PIB adhesive.

(Polymerization of Acrylic Adhesive)

A solution polymerization of 75 parts by weight of 2-ethylhexyl acrylate, 22 parts by weight of N-vinyl-2-pyrrolidone, 3 parts by weight of acrylic acid, and 0.2 parts by weight of azobisisobutyronitrile was conducted in ethyl acetate at 60°C under an inert gas atmosphere to provide a solution of an acrylic adhesive.

Each tape preparation having each composition in Tables 12 to 17 below was produced. Specifically, an antigen peptide and a cellular immunity induction promoter in amounts described in tables 12 to 17, and if desired a skin permeability enhancer and/or a pharmacologically acceptable acid, and further the pressure solution and an organic solvent (ethyl acetate, ethanol, toluene, or the like) were blended and mixed, and the mixture was spread on a release liner so that the thickness after drying was about 80 μm. The organic solvent was removed by drying, and a support was bonded thereto to prepare a tape preparation. The adhesive solution was blended so that the total amount of the respective components and the adhesive was 100 parts by weight after drying of the organic solvent. This tape preparation was cut so as to have an area of 0. 7 $cm^2$, which was used as an administration sample in immunity tests. The release liner was peeled off upon the administration.

As a support, a polyethylene terephthalate (PET) film (thickness: 25 μm) was used. As the release liner, a polyethylene terephthalate (PET) liner (thickness: 75 μm) subjected to a silicone release treatment was used. As the antigen peptide and the helper peptide, chemically synthesized and HPLC-purified products were used. The imiquimod was obtained from the same company as in the liquid formulation for external use described above. Cyclic di-GMP (c-di-GMP) and cyclic di-AMP (c-di-AMP) were purchased from Biolog Life Science Institute GmbH. Lipopolysaccharide derived from Pantoea bacterium manufactured by MACROPHI Inc., glucopyranosyl lipid manufactured by InvivoGen (MPLAs), sodium hyaluronate manufactured by Kikkoman Biochemifa Company (microhyaluronic acid FCH), ODN1826 manufactured by InvivoGen, Inc., pidotimod manufactured by Santa Cruz Biotechnology, Inc., and levamisole hydrochloride manufactured by MP Biomedicals, LLC, were used.

Mouse Immunity Test 2 (Tape preparation)

Using each of the tape preparation produced as above, a mouse immunity test was performed in the same manner as in mouse immunity test 1 described above. The number of administrations was (24 hours/week) x once, and the spleen was removed after 6 days from the administration. Further, a percentage (Th1 cell ratio) of the number of Th1 cells to the total number of the Th1 cells and the Th2 cells in a subject was measured in the same manner as in the case of the liquid formulation for external use.

In Test Example 19, the administration was performed on a skin pre-treated by ten times of tape stripping (TS) with DUNPLON tape (No.375 manufactured by Nitto Denko CS System Corporation).

With respect to a part of the tape preparations, using C57BL/6 mice, which were model animals for skin irritation evaluation, the cutaneous TSLP level after the administration to the mouse, and the transepidermal water loss of the mouse before the administration were measured in the same manner as in the case of the liquid formulation for external use described above.

The results of the immunity tests, and the measurement results of the Th1 cell ratio, the TSLP level, and the transepidermal water loss are shown in Tables 12 to 17 below together with the mice used. The "genetically modified mice" in Tables are genetically modified mice from which the cellular immunity induction owing to HLA-A* 0201 MHC-restricted peptide can be evaluated. For comparison, the results obtained from immunity caused by injection formulations described below (Reference Examples 1 to 8) were described at the end of each Table.

Table 12

| | Composition | | | | | | | TSLP (pg/mg) protein) | Physical irritation | TEWL ($g/h \cdot m^2$) | Mouse for immunological evaluation | Results of immunization (ELISPOT average number of spots) | Ratio of Th1 cells (%) |
|---|---|---|---|---|---|---|---|---|---|---|---|---|---|
| | Base | Antigen peptide | Cellular immunity induction promoter | | Skin permeabilty enhancer | Acid | Additive (Chemical irritation) | | | | | | |
| Test example 16 | PIB | MAGE3_A24 (10) | None | None | IPM (25.8) | MA(8.6) | None | 101 | None | 13 | BALB/c | 10 | unevaluable |
| Test example 17 | PIB | MAGE3_A24 (10) | IMQ(3) | None | IPM (25.8) | MA(8.6) | None | 110 | None | 13 | BALB/c | 560 | unevaluable |
| Test example 18 | PIB | MAGE3_A24 (10) | None | PEPB (1) | IPM (25.8) | MA(8.6) | None | 106 | None | 13 | BALB/c | 56 | 15 |
| Test example 19 | PIB | MAGE3_A24 (10) | None | PEPB (1) | IPM (25.8) | MA(8.6) | None | 110 | TS 10 times | 58 | BALB/c | 10 | 5 |
| Test example 20 | PIB | MAGE3_A24 (10) | IMQ(3) | PEPB(1) | IPM (25.8) | MA(8.6) | None | 143 | None | 10 | BALB/c | 963 | 25 |
| Test example 21 | PIB | MAGE3_A24 (10) | IMQ(3) | PEPB (1) | IPM (20) | MA(8.6) | BL2 (5) | 540 | None | 13 | BALB/c | 910 | |
| Test example 22 | PIB | MAGE3_A24 (10) | IMQ(3) | PEPB (1) | IPM (15) | MA(8.6) | BL2 (10) | 4200 | None | 13 | BALB/c | 675 | |
| Test example 23 | PIB | MRGE3_A24 (10) | IMQ(3) | PEPB (1) | IPM (10) | MA(8.6) | BL2 (15) | 9230 | None | 13 | BALB/c | 65 | |
| Test example 24 | PIB | MAGE3_A29 (10) | IMQ(3) | PEPB(1) | IFM (5) | MA(8.6) | BL2 (20) | 12300 | None | 13 | BALB/c | 0 | |
| Test example 25 | PIB | MAGE3_A24 (10) | c-di-GMP(cyclic dinucleotide) (1) | None | IPM (25.8) | MA(8.6) | None | 135 | None | 13 | BALB/c | 1470 | unevaluable |
| Test example 26 | PIB | MAGE3_A24 (10) | c-di-GMP(cyclic dinucleotide) (1) | PEPB(1) | IPM (25.8) | MA(8.6) | None | 140 | None | 13 | BALB/c | 1800 | 65 |
| Test example 27 | PIB | MAGE3_A24 (10) | c-di-AMP(cyclic dinucleotide) (1) | PEPB(1) | IPM (25.8) | MA(8.6) | None | | None | | BALB/c | | |

(continued)

| | Composition | | | | | | | TSLP (pg/mg protein) | Physical irritation | TEWL (g/h·m$^2$) | Mouse for immunologi cal evaluation | Results of immunization (ELISPOT average number of spots) | Ratio of Th1 cells (%) |
|---|---|---|---|---|---|---|---|---|---|---|---|---|---|
| | Base | Antigen peptide | Cellular immunity induction promoter | | Skin permeability enhancer | Acid | Additive (Chemical irritation) | | | | | | |
| Test example 28 | PIB | MAGE3_A24 (10) | lipopolysaccharide derived from Pantoea bacterium (TLR4 ligand) (1) | None | IPM (25.8) | MA(8.6) | None | 110 | None | 13 | BALB/c | 130 | unevaluable |
| Test example 29 | PIB | MAGE3_A24 (10) | lipopolysaccharide derived from Pantoea bacterium (TLR4 ligand) (1) | PEP (1) | IPM (25.8) | MA(8.6) | None | 110 | None | 13 | BALB/c | 252 | 27 |
| Test example 30 | PIB | MAGE3_A24 (10) | glucopyranosyl lipid(TLR4 ligand) (1) | PEPB(1) | IPM (25.8) | MA(8.6) | None | | None | | BALB/c | | |
| Test example 31 | PIB | MAGE3_A24 (10) | sodium hyaluronate(TLR 4 ligand) (1) | PEPB (1) | IPM (25.8) | MA(8.6) | None | | None | | BALB/c | | |
| Test example 32 | PIB | MAGE3_A24 (10) | ODN1826(TLR9 ligand) (1) | PEPB(1) | IPM (25.8) | MA(8.6) | None | | None | | BALB/c | | |
| Test example 33 | PIB | MAGE3_A24 (10) | levamisole hydrochloride(imunomodulatory small molecule drug) (1) | None | IPM (25.8) | MA(8.6) | None | 105 | None | 13 | BALB/c | 80 | unevaluable |

(continued)

| | Composition | | | | | | | TSLP (pg/mg protein) | Physical irritation | TEWL (g/h·m$^2$) | Mouse for immunologi cal evaluation | Results of immunization (ELISPOT average number of spots) | Ratio of Th1 cells (%) |
|---|---|---|---|---|---|---|---|---|---|---|---|---|---|
| | Base | Antigen peptide | Cellular immunity induction promoter | | Skin permeability enhancer | Acid | Additive (Chemical irritation) | | | | | | |
| Test example 34 | PIB | MAGE3_R24 (10) | levamisole hydrochloride(immunomodulatory small molecule drug) (1) | PEPB(1) | IPM (25.8) | MA(8.6) | None | 120 | None | 13 | BALB/c | 156 | 21 |
| Test example 35 | PIB | MAGE3_A24 (10) | pidotimod(immunomodulatory small molecule drug) (1) | None | IPM (25.8) | MA(8.6) | None | 100 | None | 13 | BALB/c | 60 | unevaluable |
| Reference example 4 | saline | MAGE3_A24 (0.125) | Montanide ISA51VG (50) | | None | None | None | | None | | BALB/c | 56 | unevaluable |

IMQ: Imiquimod (TLR7 and/or TLR8 ligand)
PIB: PIB rubber adhesive
c-di-GMP: Cyclic di-GMP
c-di-AMP: Cyclic di-AMP
IPM: Isopropyl myristate, manufactured by Croda Japan K.K.
IPP: Isopropyl palmitate, manufactured by Wako Pure Chemical Industries, Ltd.
MA: Myristic acid
SDS: Sodium dodecyl sulfate

Table 13

| | Composition | | | | | | | TSLP (pg/mg protein) | Physical irritation | TEWL (g/h·m$^2$) | Mouse for immunological evaluation | Results of immunization (ELISPOT average number of spots) | Ratio of Th1 cells (%) |
|---|---|---|---|---|---|---|---|---|---|---|---|---|---|
| | Base | Antigen peptide | Cellular imnunity induction promoter | | Skin permeability enhancer | Acid | Additive (Chemical irritation) | | | | | | |
| Test example 36 | acryl | HER2/neu E75 (10) | None | None | IPM (34.4) | None | None | 32 | None | 12 | genetically modified mouse | 5 | unevaluable |
| Test example 37 | acryl | HER2/neu E75 (10) | IMQ(3) | None | IPM (34.4) | None | None | 50 | None | 12 | genetically modified mouse | 55 | unevaluable |
| Test example 38 | acryl | HER2/neu E75 (10) | None | PEP(1) | IPM (34.4) | None | None | 35 | None | 12 | genetically modified mouse | 14 | 35 |
| Test example 39 | acryl | HER2/neu E75 (10) | IMQ(3) | PEP(1) | IPM (34.4) | None | None | 52 | None | 10 | genetically modified mouse | 78 | 67 |
| Test example 40 | acryl | HER2/neu E75 (10) | c-di-GMP(cyclic dinucleotide) (1) | None | IPM (34.4) | None | None | | None | | genetically modified mouse | | |
| Test example 41 | acryl | HER2/neu E75 (10) | c-di-GMP(cyclic dinucleotide) (1) | PEP(1) | IPM (34.4) | None | None | | None | | genetically modified mouse | | |
| Test example 42 | acryl | HER2/neu E75 (10) | c-di-AMP(cyclic dinucleotide) (1) | PEP (1) | IPM (34.4) | None | None | | None | | genetically modified mouse | | |
| Test example 43 | acryl | HER2/neu E75 (10) | lipopolysaccharide derived from Pantoea bacterium (TLR4 ligand) (1) | None | IPM (34.4) | None | None | 36 | None | 12 | genetically modified mouse | 33 | unevaluable |

(continued)

| | Composition | | | | | | | TSLP (pg/mg protein) | Physical irritation | TEWL (g/h·m$^2$) | Mouse for immunological evaluation | Results of immunization (ELISPOT average number of spots) | Ratio of Th1 cells (%) |
|---|---|---|---|---|---|---|---|---|---|---|---|---|---|
| | Base | Antigen peptide | Cellular immunity induction promoter | | Skin permeabi lity enhancer | Acid | Additive (Chemical irritation) | | | | | | |
| Test example 44 | acryl | HER2/neu E75 (10) | lipopolysaccharide derived from Pantoea bacterium (TLR4 ligand) (1) | PEP(1) | IPM (34.4) | None | None | 40 | None | 12 | genetically modified mouse | 52 | 54 |
| Test example 45 | acryl | HFR2/neu E75 (10) | glucopyranosyl lipid(TLR4 ligand) (1) | PEP(1) | IPM (34.4) | None | None | | None | | genetically modified mouse | | |
| Test example 46 | acryl | HER2/neu E75 (10) | sodium hyaluronate(TLR4 ligand) (1) | PEP(1) | IPM (34.4) | None | None | | None | | genetically modified mouse | | |
| Test example 47 | acryl | HER2/neu E75 (10) | ODN1826(TLR9 ligand) (1) | PEP(1) | IPM (34.4) | None | None | | None | | genetically modified mouse | | |
| Test example 48 | acryl | HER2/neu E75 (10) | levamisole hydrochloride(immunomodulatory small molecule drug) (1) | None | IPM (34.4) | None | None | 33 | None | 12 | genetically modified mouse | 15 | unevaluable |
| Test example 49 | acryl | HER2/neu E75 (10) | levamisole hydrochloride(immunomodulatory small molecule drug) (1) | PEP(1) | IPM (34.4) | None | None | 39 | None | 12 | genetically modified mouse | 32 | 43 |

(continued)

| | Composition | | | | | | | TSLP (pg/mg protein) | Physical irritation | TEWL (g/h·m$^2$) | Mouse for immunological evaluation | Results of immumization (ELISPOT average number of spots) | Ratio of Th1 cells (%) |
|---|---|---|---|---|---|---|---|---|---|---|---|---|---|
| | Base | Antigen peptide | Cellular immunity induction promoter | | Skin permeabi lity enhancer | Acid | Additive (Chemical irritation) | | | | | | |
| Test example 50 | acryl | HER2/neu E75 (10) | pidotimod(immunomodulatory small molecule drug) (1) | None | IPM (34.4) | None | None | 31 | None | 12 | genetically modified mouse | 14 | unevaluable |
| Test example 51 | acryl | HER2/neu E75 (10) | pidotimod (immmomodulatory small molecule drug) (1) | FEP(1) | IPM (34.4) | None | None | 35 | None | 12 | genetically modified mouse | 30 | 41 |
| Test example 52 | acryl | HER2/neu E75 (10) | IMQ(3) | PADRE (1) | IPP (34.4) | None | None | 48 | None | 12 | genetically modified mouse | 62 | 68 |
| Test example 53 | acryl | HER2/neu E75 (10) | IMQ(3) | PEP(1) | None | None | None | 42 | None | 12 | genetically modified mouse | 35 | 44 |
| Test example 54 | PIB | HER2/neu E75 (10) | IMQ(3) | PEP(1) | IPM (34.4) | None | None | 25 | None | 10 | genetically modified mouse | 23 | 49 |
| Reference example 5 | saline | HER2/neu E75 (0.125) | Montanide ISA51VG (50) | | None | None | None | | None | | genetically modified mouse | 110 | unevaluable |

Acryl: Acrylic adhesive
PADRE: Universal helper peptide (SEQ NO: 15)

Table 14

| | Composition | | | | | | | TSLP (pg/mg protein) | Physical irritation | TEWL (g/h·m$^2$) | Mouse for immunological evaluation | Results of immunization (ELISPOT average number of spots) | Ratio of Th1 cells (%) |
|---|---|---|---|---|---|---|---|---|---|---|---|---|---|
| | Base | Antigen peptide | Cellular immunity induction promoter | | Skin permeability enhancer | Acid | Additive (Chemical irritation) | | | | | | |
| Test example 55 | PIB | HER2/neu A02 (10) | lipopolysaccharide derived from Pantoea bacterium (TLR4 ligand) (1) | None | IPM (25.8) | MA(8.6) | None | 95 | None | 12 | genetically modified mouse | 52 | unevaluable |
| Test example 56 | PIB | HER2/neu A02 (10) | IMQ(3) | PEP(1) | IPM (25.8) | MA(8.6) | None | 112 | None | 10 | genetically modified mouse | 111 | 72 |
| Reference example 7 | saline | HER2/neu A02 (0.125) | Montanide ISA51VG (50) | | None | None | None | | None | | genetically modified mouse | 93 | unevaluable |

Table 15

| | Composition | | | | | | | TSLP (pg/mg protein) | Physical irritation | TEWL (g/h·m$^2$) | Mouse for immunological evaluation | Results of immunization (ELISPOT average number of spots) | Ratio of Th1 cells (%) |
|---|---|---|---|---|---|---|---|---|---|---|---|---|---|
| | Base | Antigen peptide | Cellular immunity induction promoter | | Skin permeability enhancer | Acid | Additive (Chemical irritation) | | | | | | |
| Test example 57 | acryl | IPEP87 (10) | lipopolysacharide derived from Pantoea bacterium (TLR4 ligand) (1) | None | IPM (25.8) | MA(8.6) | None | 90 | None | 12 | genetically modified mouse | 67 | unevaluable |
| Test example 58 | acryl | IPEP87 (10) | IMQ(3) | PEP(1) | IPM (25.8) | MA(8.6) | None | 95 | None | 10 | genetically modified mouse | 132 | 85 |
| Test example 59 | PIB | IPEP87 (10) | IMQ(3) | PEE(1) | IPM (25.8) | MA(8.6) | None | 77 | None | 10 | genetically modified mouse | 227 | 60 |

Table 16

| | Composition | | | | | | | TSLP (pg/mg protein) | Physical irritation | TEWL ($g/hm^2$) | Mouse for immunological evaluation | Results of immunization (ELISPOT average number of spots) | Ratio of Th1 cells (%) |
|---|---|---|---|---|---|---|---|---|---|---|---|---|---|
| | Base | Antigen peptide | Cellular immunity induction promoter | | Skin permeability enhancer | Acid | Additive (Chemical irritation) | | | | | | |
| Test example 60 | PIB | PR1 (10) | lipopolysaccharide derived from Pantoea bacterium (TLR4 ligand) (1) | None | IPM (25.8) | MA(8.6) | None | 92 | None | 12 | genetically modified mouse | 29 | unevaluable |
| Test example 61 | PIB | PR1 (10) | IMQ (3) | PEP (1) | IFM (25.8) | MR(8.6) | None | 89 | None | 10 | genetically modified mouse | 33 | 48 |

Table 17

| | Composition | | | | | | | TSLP (pg/mg protein) | Physical irritation | TEWL (g/h·m$^2$) | Mouse for immunological evaluation | Results of immunization (ELISPOT average number of spots) | Ratio of Th1 cells (%) |
|---|---|---|---|---|---|---|---|---|---|---|---|---|---|
| | Base | Antigen peptide | Cellular immunity induction promoter | | Skin permeability enhancer | Acid | Additive (Chemical irritation) | | | | | | |
| Test example 62 | acryl | MAGE3_A02 (10) | lipopolysaccharide derived from Pantoea bacterium(TLR4 ligand) (1) | None | IPM (35.2) | None | None | 65 | None | 12 | genetically modified mouse | 55 | unevaluable |
| Test example 63 | acryl | MAGE3_A02 (10) | IMQ (3) | PEP (1) | IFM(35.2) | None | None | 69 | None | 10 | genetically modified mouse | 97 | 83 |

Cream Formulation

Each cream formulation having each composition shown in Tables 19 to 24 and 26 below was produced. Specifically, an antigen (peptide or protein), a cellular immunity induction promoter other than a helper peptide, and the helper peptide in an amount (parts by weight) set forth in Tables 19 to 24 and 26, 15 parts by weight of DMSO, and if desired, and an additive were blended, to which a base material (base cream) was added so that the total amount was 100 parts by weight, and the resultant was mixed to provide a cream formulation. With respect to each of the cream formulations of Test Examples, where the amount blended was specified in Tables 19 to 24 and 26, the amount of each component blended was as shown in the Tables. The base cream used was one prepared by blending and mixing materials in composition described in Table 18.

A composite base material, in which a laminate of a PET film/a PET non-woven fabric (area: 0.7 $cm^2$) was bonded to the central part of an adhesive tape and the PET film was attached to the tape, was prepared. The composite base material in which the non-woven fabric part was coated with 4 mg of the cream formulation was used as an administration sample for immunity tests.

Mouse Immunity Test 3 (Cream Formulation)

Using each of the cream formulations produced as above, mouse immunity test was performed in the same manner as in mouse immunity test 1. The number of administrations was (24 hours/week) x once, and the spleen was removed after 6 days from the administration. Further, a percentage (Th1 cell ratio) of the number of Th1 cells to the total number of the Th1 cells and the Th2 cells in a subject was measured in the same manner as in the case of the liquid formulation for external use.

In Test Examples 65, 67, 74, 78 and 79, the administration was performed on a skin pre-treated by ten times of tape stripping (TS) with DUNPLON tape (No. 375 manufactured by Nitto Denko CS System Corporation), and in Test Example 71, the administration was performed on a skin injured with a micro cutter (MICRO FEATHER No.7330G manufactured by FEATHER).

With respect to a part of the cream formulations, using C57BL/6 mice, which were model animals for skin irritation evaluation, the cutaneous TSLP level after the administration to the mouse, and the transepidermal water loss of the mouse before the administration were measured in the same manner as in the case of the liquid formulation for external use described above.

The results of the immunity tests, and the measurement results of the Th1 cell ratio, the TSLP level, and the transepidermal water loss are shown in Tables 19 to 24 and 26 below together with the mice used. For comparison, the results obtained from immunity caused by injection formulations described below (Reference Examples 1 to 8) were described at the end of each Table.

Table 18

| | Base cream |
|---|---|
| White petrolatum | 60.7% by weight |
| Sorbitan monostearate | 0.7% by weight |
| Isostearic acid | 12.0% by weight |
| Benzyl alcohol | 2.4% by weight |
| Cetanol | 2.4% by weight |
| Stearyl alcohol | 3.5% by weight |
| Polysorbate 60 | 3.5% by weight |
| Concentrated glycerol | 2.4% by weight |
| Water | 12.4% by weight |

The white petrolatum, sorbitan monostearate, isostearic acid, benzyl alcohol, stearyl alcohol, Polysorbate 60, concentrated glycerol, and dimethylsulfoxide (DMSO) were purchased from Wako Pure Chemical Industries, Ltd. The cetanol was purchased from Tokyo Chemical Industry Co., Ltd. The OVA protein was purchased from Sigma-Aldrich Corporation. As the KLH-derived peptide (TFA salt), Peptide-25B (Pep25B), antigen peptide, and helper peptide, chemically synthesized and HPLC-purified products were used. The imiquimod was obtained from the same company as in the liquid formulation for external use described above.

Table 19

| | Composition | | | | | TSLP (pg/mg protein ) | Physical irritation | TEWL (g/h·m$^2$ ) | Mouse for immunological evaluation | Results of immunization (ELISPOT average number of spots) | Ratio of Th1 cells (%) |
|---|---|---|---|---|---|---|---|---|---|---|---|
| | Base | Antigen peptide | Cellular immunity induction promoter | | Additive (Chemical irritation) | | | | | | |
| Test example 64 | base cream | HER2/neu_A2 4 (5) | None | PEPB (0.3) | None | 63 | None | 10 | BALB/c | 138 | 36 |
| Test example 65 | base cream | HER2/neu_A2 4 (5) | None | PEPB (0.3) | None | 64 | TS 10 times | 58 | BALB/c | 10 | 5 |
| Test example 66 | base cream | HER2/neu_A2 4 (5) | None | PEPB (0.3) | BL2 (content: 20%) | 780 | None | 10 | BALB/c | 19 | 9 |
| Test example 67 | base cream | HER2/neu_A2 4 (5) | IMQ (3) | PEPB (0.3) | None | 59 | TS 10 times | 58 | BALB/c | 71 | 29 |
| Test example 68 | base cream | HER2/neu_A2 4 (5) | IMQ (3) | PE PB (0.3) | None | 81 | None | 10 | BALB/c | 1067 | 44 |
| Reference example 3 | saline | HER2/neu_A2 4 (0.125) | Montanide ISA51VG (50) | | None | | None | | BALB/c | 234 | unevaluable |

Table 20

| | Composition | | | | | TSLP (pg/mg protein) | Physical irradiation | TEWL ($g/h \cdot m^2$) | Mouse for immunological evaluation | Results of immunization (ELISPOT average number of spots) | Ratio of Th1 cells (%) |
|---|---|---|---|---|---|---|---|---|---|---|---|
| | Base | Antigen peptide | Cellular immunity induction promoter | | Additive (Chemical irritation) | | | | | | |
| Test example 69 | base cream | OVA protein (2.5) | IMQ (3) | None | None | 61 | None | 10 | BALB/c | 270 | 66 |

Table 21

| | Composition | | | | | TSLP (pg/mg protein) | Physical irritation | TEWL (g/h·$m^2$) | Mouse for immunological evaluation | Results of immunization (ELISPOT average number of spots) | Ratio of Th1 cells (%) |
|---|---|---|---|---|---|---|---|---|---|---|---|
| | Base | Antigen peptide | Cellular immunity induction promoter | | Additive (Chemical irritation) | | | | | | |
| Test example 70 | base cream | KLH-derived peptide(2) | IMQ (3) | PEP (0.3) | None | 80 | None | 10 | C57BL/6 | 165 | 75 |

Table 22

| | Composition | | | | | TSLP (pg/mg protein) | Physical irritation | TEWL (g/h·m$^2$) | Mouse for immunological evaluation | Results of immunization (ELISPOT average number of spots) | Ratio of Th1 cells (%) | % Specific Lysis (In vivo CTL assay) |
|---|---|---|---|---|---|---|---|---|---|---|---|---|
| | Base | Antigen peptide | Cellular immunity induction promoter | | Additive (Chemical irritation) | | | | | | | |
| Test example 71 | base cream | HER2/neu_E75 (25) | IMQ (3) | PEP (0.3) | None | 67 | microcutter | 66 | genetically modified | 73 | 46 | |
| Test example 72 | base cream | HER2/neu_E75 (25) | IMQ (3) | PEP (0.3) | None | 78 | None | 10 | genetically modified | 123 | 64 | 30 |
| Reference example 5 | saline | HER2/neu E75 (0.125) | Montanide ISA51VG (50) | | None | | None | | genetically modified mouse | 110 | unevaluable | 28 |

Table 23

| | Composition | | | | | TSLP (pg/mg protein) | Physical irritation | TEWL ($g/h \cdot m^2$) | Mouse for immunological evaluation | Results of immunization (ELISPOT average number of spots) | Ratio of Th1 cells (%) |
|---|---|---|---|---|---|---|---|---|---|---|---|
| | Base | Antigen peptide | Cellular immunity induction promoter | | Additive (Chemical irritation) | | | | | | |
| Test example 73 | base cream | MAGE3_A24 (5) | None | PEPB (0.3) | None | 71 | None | 10 | BALB/c | 149 | 32 |
| Test example 74 | base cream | MAGE3_A24 (5) | None | PEPB (0.3) | None | 60 | TS 10 times | 58 | BALB/c | 3 | 4 |
| Test example 75 | base cream | MAGE3_A24 (5) | None | PEPB (0.3) | BL2 (content: 20%) | 810 | None | 10 | BALB/c | 5 | 7 |
| Test example 76 | base cream | MAGE3_A24 (5) | IMQ (3) | PEPB (0.3) | None | 81 | None | 10 | BALB/c | 326 | 43 |
| Reference example 4 | saline | MAGE3_A24 (0.125) | Montanide ISA51VG (50) | | None | | None | | BALB/c | 56 | unevaluable |

Table 24

| | Composition | | | | | TSLP (pg/mg protein) | Physical irritation | TEWL ($g/h \cdot m^2$) | Mouse for immunological evaluation | Results of immunization (ELISPOT average number of spots) | Ratio of Th1 cells (%) |
|---|---|---|---|---|---|---|---|---|---|---|---|
| | Base | Antigen peptide | Cellular immunity induction promoter | | Additive (Chemical irritation) | | | | | | |
| Test example 77 | base cream | survivin 2B (5) | None | PEPB (0.3) | None | 72 | None | 10 | BALB/c | 329 | 27 |
| Test example 78 | base cream | survivin 2B (5) | None | PEPB (0.3) | None | 65 | TS 10 times | 58 | BALB/c | 2 | 8 |
| Test example 79 | base cream | survivin 2B (5) | IMQ (3) | PEPB (0.3) | None | 54 | TS 10 times | 58 | BALB/c | 99 | 21 |
| Test example 80 | base cream | survivin 2B (5) | IMQ (3) | PEPB (0.3) | None | 81 | None | 10 | BALB/c | 400 | 33 |
| Reference example 1 | saline | survivin 2B (0.125) | Montanide ISA51VG (50) | | None | | None | | BALB/c | 313 | unevaluable |

Subcutaneous Injection Formulation

Each subcutaneous injection formulation having each composition shown in Table 25 described below, which was used as an administration sample in immunity tests, was prepared. Specifically, to an antigen peptide having an amount set forth in Table 25 and Montanide ISA51VG (Freund Corporation) which was an adjuvant were added 0.5 parts by weight of an additive (DMSO) and saline which was a base material so that the total amount was 100 parts by weight, and the resultant was mixed in a homogenizer to prepare an injection formulation. As the antigen peptide, a chemically synthesized HPLC-purified product was used.

Mouse Immunity Test 4 (Injection Formulation)

Using each of the injection formulations produced as above, a mouse immunity test was performed in the same manner as in mouse immunity test 1 described above. The dosage was 200 μL, the number of administrations was one, and the spleen was removed after 6 days from the administration. The results of the immunity test are shown in Table 25 below together with the mice used.

Table 25

| | Composition | | | | Physical irritation | Mouse for immunological evaluation | Results of immunization (ELISPOT average number of spots) | Ratio of Th1 cells (%) |
|---|---|---|---|---|---|---|---|---|
| | Base | Antigen peptide | Cellular immunity induction promoter | Additive (Chemical irritation) | | | | |
| Reference Example 1 | Saline | Survivin 2B (0.125) | Montanide ISA51VG(50) | None | None | BALB/c | 313 | Unevaluable |
| Reference Example 2 | Saline | GPC3 (0.125) | Montanide ISAS1VG(50) | None | None | BALS/c | 1.0 | Unevaluable |
| Reference Example 3 | Saline | HER2/neu_A24 (0.125) | Montanide ISA51VG(50) | None | None | BALB/c | 234 | Unevaluable |
| Reference Example 4 | Saline | MAGE3_A24 (0.125) | Montanide ISA51VG(50) | None | None | BALB/c | 56 | Unevaluable |
| Reference Example 5 | Saline | HER2/neu E75 (0.125) | Montanide ISA51VG(50) | None | None | Genetically modified mice | 110 | Unevaluable |
| Reference Example 6 | Saline | PR1 (0.125) | Montanide ISA51VG(50) | None | None | Genetically modified mice | 144 | Unevaluable |
| Reference Example 7 | Saline | HER2/neu_A02 (0.125) | Montanide ISA51VG(50) | None | None | Genetically modified mice | 93 | Unevaluable |
| Reference Example 8 | Saline | OVA peptide (0.4) | Montanide ISA51VG (50) | None | None | C57BL/6 | 30 | Unevaluable |

In vivo CTL assay

Seven days after final immunization, the spleen cells (target cell or control cell) were transplanted according to the following procedure, and then, the spleen was isolated after 18 hours. The % Specific Lysis was obtained by performing the FACS measurement.

Procedure 1. Collection of spleen cells of naïve mouse

Naive mouse (C57BL/6) was used. Spleen was isolated from the naive mouse and mashed using a glass slide in a petri dish containing RPMI1640 medium. The mashed spleen was put into a 50 mL tube and centrifuged at 10°C and 1100 rpm for 5 minutes. The supernatant was discarded. 20 mL of Lysis Buffer was added to the tube, followed by incubation at room temperature for 5 minutes. 20 mL of the medium was added to the tube and the tube was then centrifuged. The medium was added to the tube and the resultant was passed through a cell strainer to give spleen cell suspension.

Procedure 2. Labeling of the spleen cells with the antigen

The spleen cells prepared in Procedure 1 were centrifuged at 10°C and 1100 rpm for 5 minutes, the supernatant was discarded, and HBSS buffer was added to give cell suspension of $2 \times 10^7$ cells/mL. The cell suspension was dispensed into two 50 mL tubes, 100 μM of the antigen solution (the antigen was the same antigen used in the immunization test) was added to one of the tubes containing the cell solution so that the final concentration became 10 μM, to obtain a target cell. The cell in another tube was adopted as control. The cells in both tubes were incubated at 37°C for 1 hour, centrifuged, the supernatant was discarded, and a medium was added.

Procedure 3. Labeling of the spleen cells with CFSE

The cell labelled with the antigen according to Procedure 2 was centrifuged, and 0.1% BSA-PBS was added to $1 \times 10^7$ cells/mL. To the target cell suspension was added 5 mM CFSE solution to give the final concentration of 10 μM, and to the control cell suspension was added 5 mM CFSE solution to give the final concentration of 1 μM, and the mixture was vortexed, followed by incubation at 37°C for 10 minutes. Thereafter, centrifugation was performed, the supernatant was discarded, and the medium was added.

Procedure 4. Transplantation of spleen cell

The cell labelled with CFSE according to Procedure 3 was centrifuged, the supernatant was discarded, and HBSS buffer was added to the cells to give cell suspension of $5 \times 10^7$ cells/mL. Equal amounts of the target cell suspension and the control cell suspension were mixed, and 200 μL aliquot of the mixture was introduced into each immunized mouse via orbital veins (transplanted cell number: $1 \times 10^7$ cells/animal).

Procedure 5. Preparation of spleen cell of the immunized mouse and measurement of FACS

Eighteen hours after the transplantation of the spleen cells, spleen of the mouse was isolated, and spleen cell suspension was prepared in the same manner as in Procedure 1. Thereafter, CFSE-positive cells were detected by FACS, and the ratio between CFSE high cells (target cells) and CFSE low cells (control cells) was obtained. The cytotoxic activity was calculated by the formula shown below. The obtained value can be used as an index showing the ability of the antigen specific killer cells induced by the immunization with the vaccine composition to attack specifically the cells that present the antigen in the living body. It was confirmed that the composition of the present invention can induce strong antigen-specific cellular immunity.

$$r = (\%\ \text{CFSE low cells})/(\%\ \text{CFSE high cells})$$

$$\%\ \text{Specific Lysis} = (1 - (r_{\text{non immunized}}/r_{\text{immunized}})) \times 100$$

Cancer-Bearing Mouse Test

C57BL/6 mice were inoculated with OVA-expressing E.G7 cancer cells (E.G7) (purchased from ATCC) through subcutaneous injection ($2 \times 10^6$ cells/mouse). After that, immunization was performed 5 times every 3 to 4 days, and a size of a tumor was evaluated at the 25th day from the inoculation of the cancer cells. The effect of the immunity induced was evaluated by the inhibition of the tumor growth, and it was confirmed that the preparation administered of the present invention had a high effect.

Table 26

| | Composition | | | | | TSLP (pg/mg protein) | Physical irritation | TEWL, (g/h·m$^2$) | Mouse for immunological evaluation | Results of immunization (ELISPOT average number of spots) | Ratio of Th1 cells (%) | % Specific Lysis (In vivo CTL assay) | Tumor cell size (nm$^3$) (cancer-bearing mouse test) |
|---|---|---|---|---|---|---|---|---|---|---|---|---|---|
| | Base | Antigen peptide | Cellular immunity induction promoter | | Additive (Chemical irritation) | | | | | | | | |
| Test example 81 | base cream | OVA peptide (5) | None | None | None | | None | 10 | C57BL/6 | 18 | unevaluable | 8 | 8000 |
| Test example 82 | base cream | OVA peptide (5) | IMQ (3) | PADRE (1) | None | | None | 10 | C57BL/6 | 153 | 52 | 30 | 6500 |
| Test example 83 | base cream | OVA peptide (5) | c-di-G1P (cyclic dinucleotide) (1) | None | None | | None | 10 | C57BL/6 | 1055 | unevaluable | | |
| Test example 84 | base cream | OVA peptide (5) | c-di-GMP (cyclic dinucleotide) (1) | PADRE (1) | None | | None | 10 | C57BL/6 | 1500 | 75 | 85 | 4200 |
| Reference example 8 | saline | OVA peptide (0.4) | Montanide ISA51VG (50) | | None | | None | | C57BL/6 | 30 | | | 8000 |

OVA peptide: OVA peptide (the peptide consisting of 8 amino acids having the following sequence: Ser-Ile-Ile-Asn-Phe-Glu-Lys-Leu; the peptide chemically synthesized and HPLC purified was used.)

As shown in Table 3 (Test Examples 6 and 7), Table 12 (Test Examples 18 and 19), Table 19 (Test Examples 64 to 65 and 67 to 68), Table 22 (Test Examples 71 and 72), Table 23 (Test Examples 73 and 74) and Table 24 (Test Examples 77 to 80), the TEWL values were increased, the ratio of Th1 cells was decreased and the level of immunity induction (ELISPOT) was decreased by the tape stripping treatment or the micro cutter treatment. For these results, it was found that the degree of the physical irritancy to the skin before the administration can be determined using the degree of the TEWL of the skin before the administration as the indicator. It was found that a strong physical irritation to the skin causes a negative effect on the induction of immunity.

As shown in Table 3 (Test Examples 3 to 5 and 7), Table 12 (Test Examples 20 to 24), Table 19 (Test Examples 64 and 66) and Table 23 (Test Examples 73 and 75), when the liquid formulation for external use comprising a large amount of oleyl alcohol with high skin irritancy or the tape preparation or cream formulation comprising the surfactant BL2 with high skin irritancy was used, the TSLP production was highly induced, the ratio of Th1 cells was decreased and the level of immunity induction (ELISPOT) was decreased. For these results, it was found that the degree of the chemical irritancy to the skin caused by a drug to be administered can be determined using the degree of the TSLP level in the skin after the administration as the indicator. It was revealed that a strong chemical irritation to the skin also causes a negative effect on the induction of immunity.

As shown in Tables 12, 13, 19, 23, 24 and 26, the induction of cellular immunity by transdermal administration of an antigen was enhanced by using a specific cellular immunity induction promoter in combination with the antigen. Specifically, when a vaccine composition is transdermally administered, a high cellular immunity inducing effect was obtained by using one or more cellular immunity induction promoters selected from the group consisting of TLR ligand (based on the comparisons between Test example 16 and Test example 17 or 28, between Test example 18 and Test example 20 or 29, between Test example 36 and Test example 37 or 43, between Test example 38 and Test example 39 or 44, between Test example 64 and Test example 68, between Test example 73 and Test example 76, and between Test example 77 and Test example 80), a cyclic dinucleotide (based on the comparisons between Test example 16 and Test example 25, between Test example 18 and Test example 26, and between Test example 81 and Test example 83), a helper peptide (based on the comparisons between Test example 16 and Test example 18, and between Test example 36 and Test example 38) and an immunomodulatory small molecule drug (based on the comparisons between Test example 16 and Test example 33 or 35, between Test example 18 and Test example 34, between Test example 36 and Test example 48 or 50, and between Test example 38 and Test example 49 or 51).

In transdermal immunization tests performed under different skin irritation conditions, or immunization tests in which a vaccine composition comprising a cellular immunity induction promoter is used, a relationship between the level of cellular immunity induction and the ratio of Th1 cells was studied. It was found that a strong cellular immunity is induced when the ratio of Th1 cells is 10% or more.

From the results of the tests, it was found that the cellular immunity is effectively induced by the transdermal administration of the vaccine composition of the present invention as compared with the injection administration thereof.

## Claims

1. A vaccine composition comprising an antigen for transdermal administration for use in the induction of cellular immunity, wherein transdermal administration of the composition provides a Th1 cell ratio in a model animal for immunological evaluation administered with the composition of 10% or more.

2. The vaccine composition according to claim 1, wherein the composition is for use in the treatment of a cancer.

3. The vaccine composition according to claim 1, wherein the composition is for use in the treatment of a viral disease.

4. The vaccine composition according to any one of claims 1-3, wherein the composition comprises at least one cellular immunity induction promoter selected from the group consisting of a TLR ligand, a cyclic dinucleotide, a helper peptide and an immunomodulatory small molecule drug.

5. The vaccine composition according to claim 4, wherein the cellular immunity induction promoter is a helper peptide.

6. The vaccine composition according to claim 4, wherein the cellular immunity induction promoter is a combination of a helper peptide and at least one substance selected from the group consisting of a TLR ligand, a cyclic dinucleotide and an immunomodulatory small molecule drug.

7. The vaccine composition according to any one of claims 1 to 6, which is administered under a mildly irritating condition.

8. The vaccine composition according to claim 7, wherein the mildly irritating condition is a condition under which transepidermal water loss (TEWL) in a model animal for skin irritation evaluation before the administration of the composition is 50 $g/h \cdot m^2$ or less.

9. The vaccine composition according to claim 7 or 8, wherein the mildly irritating condition is a condition under which the cutaneous TSLP level in a model animal for skin irritation evaluation at completion of the administration of the composition is 10000 pg/mg protein or less.

10. 10 The vaccine composition according to any one of Claims 1-9, wherein the antigen is a peptide selected from the group consisting of survivin-2B peptide and/or modified survivin-2B peptide, GPC3 peptide and/or modified GPC3 peptide, HER2/neu_A24 peptide and/or modified HER2/neu_A24 peptide, MAGE3_A24 peptide and/or modified MAGE3_A24 peptide, IPEP87 peptide and/or modified IPEP87 peptide, PR1 peptide and/or modified PR1 peptide, HER2/neu_A02 peptide and/or modified HER2/neu_A02 peptide, MAGE3_A02 peptide and/or modified MAGE3_A02 peptide, HBVenv peptide and/or modified HBVenv peptide, and MUC1 peptide and/or modified MUC1 peptide.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20080193487 A
- JP 2002531415 A
- US 20080112974 A
- JP 7505883 A
- JP 2007529531 A
- JP 4422903 B
- WO 200006602 A
- WO 2005095598 A
- WO 2007097358 A
- WO 2008081701 A
- WO 9850399 A
- US 6303347 B
- US 6764840 B


### Non-patent literature cited in the description


- **HOSOI AKIHIRO et al.** *Cancer Research,* 2008, vol. 68, 3941-3949
- **ZHENGRONG CUI et al.** *Pharmaceutical Research,* 2002, vol. 19 (7), 947-953
- **KAWAI et al.** *Nucleic Acids Research,* vol. 3, 103-4
- Japanese Pharmacopoeia